(19) Europäisches Patentamt
European Patent Office
Office européen des brevets

(11) **EP 3 967 676 A1**

(12) **EUROPEAN PATENT APPLICATION**
published in accordance with Art. 153(4) EPC

(43) Date of publication:
**16.03.2022 Bulletin 2022/11**

(21) Application number: **20802015.6**

(22) Date of filing: **27.04.2020**

(51) International Patent Classification (IPC):
*C07C 47/058* (2006.01)   *C07C 47/58* (2006.01)
*C12P 7/22* (2006.01)   *C12P 7/24* (2006.01)
*C12N 1/00* (2006.01)

(52) Cooperative Patent Classification (CPC):
**C07C 47/58; C12N 1/00; C12P 7/22; C12P 7/24**

(86) International application number:
**PCT/JP2020/017952**

(87) International publication number:
**WO 2020/226087 (12.11.2020 Gazette 2020/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.05.2019   JP 2019088499**
**08.05.2019   JP 2019088500**

(71) Applicant: **AJINOMOTO CO., INC.**
**Chuo-ku**
**Tokyo 104-8315 (JP)**

(72) Inventors:
• **OTA, Naohiro**
**Kawasaki-shi, Kanagawa 210-8681 (JP)**
• **OKASORA, Takahiro**
**Kawasaki-shi, Kanagawa 210-8681 (JP)**
• **MORI, Kenichi**
**Kawasaki-shi, Kanagawa 210-8681 (JP)**
• **INAGAKI, Tomohiro**
**Kawasaki-shi, Kanagawa 210-8681 (JP)**

(74) Representative: **Hoffmann Eitle**
**Patent- und Rechtsanwälte PartmbB**
**Arabellastraße 30**
**81925 München (DE)**

(54) **VANILLIN PRODUCTION METHOD**

(57)   Provided is a method for collecting an objective substance such as vanillin from a fermentation broth. Upon collecting an objective substance from a fermentation broth containing the objective substance by solvent extraction using an organic solvent, emulsification during the solvent extraction can be prevented by treating the fermentation broth with a protease and then submitting it to the solvent extraction, or by carrying out the solvent extraction with an agitation power adjusted to a predetermined range, and thereby the objective substance can be collected from the fermentation broth.

EP 3 967 676 A1

**Description**

Technical Field

The present invention relates to a method for collecting an objective substance such as vanillin from a fermentation broth.

Background Art

Collection of substances from an aqueous layer such as a fermentation broth can be carried out by, for example, solvent extraction using an organic solvent. However, it is known that emulsification is induced between an aqueous layer and an organic layer during solvent extraction from a fermentation broth containing various impurities, and thereby the separability between the layers may be reduced (Patent Documents 1 to 2). Specifically, for example, it is known that it is known that, during solvent extraction of lactic acid from a lactic acid fermentation broth, the separability between aqueous and organic layers is reduced due to contaminated proteins in the fermentation broth, and it has been proposed to remove the contaminated proteins prior to the solvent extraction (Patent Document 1).

Prior art references

Patent documents

Patent document 1: JP2011-177159A
Patent document 2: WO2010/114552

Summary of the Invention

Object to be Achieved by the Invention

An object of the present invention is to provide a method for collecting an objective substance such as vanillin from a fermentation broth.

Means for Achieving the Object

In order to achieve the aforementioned object, the inventors of the present invention conducted various researches. As a result, they found that, upon collecting vanillin from a fermentation broth containing vanillin by solvent extraction using an organic solvent, emulsification during the solvent extraction can be prevented by treating the fermentation broth with a protease and then submitting it to the solvent extraction, or by carrying out the solvent extraction with an agitation power adjusted to a predetermined range, and accomplished the present invention.
The present invention can be thus embodied, for example, as follows.

[1] A method for producing an objective substance, the method comprising the following steps (1A) and (1B):

(1A) a step of treating a fermentation broth containing the objective substance with a protease; and
(1B) a step of extracting the objective substance with an organic solvent from the fermentation broth after the treatment,
wherein the protease is one or more kinds of proteases selected from proteases derived from *Bacillus* bacteria and proteases derived from *Aspergillus* fungi.

[2] The method mentioned above, wherein the objective substance is an aromatic aldehyde.

[3] The method mentioned above, wherein the objective substance is vanillin.

[4] The method mentioned above, provided that cases where the protease is Protease M "Amano" SD, Protease A "Amano" SD, or Protease P "Amano" 3SD are excluded.

[5] The method mentioned above, wherein the protease is one or more kinds of proteases selected from serine proteases and metalloproteases.

[6] The method mentioned above, wherein the protease is one or more kinds of proteases selected from subtilisin and bacillolysin.

[7] The method mentioned above, wherein the protease is one or more kinds of proteases selected from proteases derived from *Bacillus licheniformis,* proteases derived from *Bacillus amyloliquefaciens,* and proteases derived from *Aspergillus oryzae.*

[8] The method mentioned above, wherein the protease is one or more kinds of proteases selected from Alcalase (registered trademark) 2.4L FG, Protin SD-NY10, Protin SD-AY10, and ProteAX (registered trademark).

[9] The method mentioned above, wherein the use amount of the protease is 10 to 500,000 U in terms of the protease activity measured by the Folin method, 0.2 to 10,000 U in terms of the protease activity measured by the LNA method, or 0.5 to 20,000 mAU in terms of the protease activity measured by the Anson method, to 100 mL of the fermentation broth.

[10] The method mentioned above, wherein the organic solvent is one or more kinds of components selected from toluene, ethyl acetate, and butyl acetate.

[11] The method mentioned above, wherein the fermentation broth in the step (1A) is a fermentation broth after cell removal.

[12] The method mentioned above, wherein the step (1B) is carried out under stirring conditions.

[13] The method mentioned above, wherein the method further comprises, prior to the step (1A), a step of generating the objective substance using a microorganism having an objective substance-producing ability, to thereby obtain the fermentation broth.

[14] The method mentioned above, wherein the method further comprises, after the step (1B), collecting an organic layer containing the objective substance.

[15] The method mentioned above, wherein the method further comprises purifying the objective substance from the organic layer.

[16] A method for producing an objective substance, the method comprising the following step (2A):
(2A) a step of extracting the objective substance with an organic solvent from a fermentation broth containing the objective substance,
wherein the step (2A) is carried out by stirring the fermentation broth and the organic solvent with an adjusted agitation power.

[17] The method mentioned above, wherein the objective substance is an aromatic aldehyde.

[18] The method mentioned above, wherein the objective substance is vanillin.

[19] The method mentioned above, wherein the adjusted agitation power is an agitation power with which a liquid-liquid interface is maintained.

[20] The method mentioned above, wherein the adjusted agitation power is more than 0 and 20 W/kL or less.

[21] The method mentioned above, wherein the adjusted agitation power is more than 0 and 15 W/kL or less.

[22] The method mentioned above, wherein the adjusted agitation power is more than 0 and 10 W/kL or less.

[23] The method mentioned above, wherein the adjusted agitation power is 1.5 W/kL or more.

[24] The method mentioned above, wherein the organic solvent is one or more kinds of components selected from toluene, ethyl acetate, and butyl acetate.

[25] The method mentioned above, wherein the organic solvent is toluene, and the adjusted agitation power is more than 0 and 20 W/kL or less.

[26] The method mentioned above, wherein the organic solvent is ethyl acetate, and the adjusted agitation power is more than 0 and 10 W/kL or less.

[27] The method mentioned above, wherein the fermentation broth is a fermentation broth containing cells.

[28] The method mentioned above, wherein the method further comprises, prior to the step (2A), a step of generating the objective substance using a microorganism having an objective substance-producing ability, to thereby obtain the fermentation broth.

[29] The method mentioned above, wherein the method further comprises, after the step (2A), collecting an organic layer containing the objective substance.

[30] The method mentioned above, wherein the method further comprises purifying the objective substance from the organic layer.

Brief Description of Drawings

[FIG. 1] A diagram (photograph) showing emulsification-preventing effect of treatment with protease (Alcalase 2.4L FG) on solvent extraction of vanillin with toluene.
[FIG. 2] A diagram (photograph) showing degradation of contaminated proteins in vanillin fermentation broth by treatment with protease (Alcalase 2.4L FG).
[FIG. 3] A diagram (photograph) showing emulsification-preventing effect of treatment with protease (Alcalase 2.4L FG) on solvent extraction of vanillin with ethyl acetate or butyl acetate.
[FIG. 4] A diagram (photograph) showing emulsification-preventing effect of treatment with various proteases on solvent extraction of vanillin with butyl acetate.
[FIG. 5] A diagram showing effect of agitation power on yield and extraction time during solvent extraction of vanillin from fermentation broth using toluene.
[FIG. 6] A diagram showing effect of agitation power on yield and mass transfer coefficient during solvent extraction of vanillin from fermentation broth using toluene.
[FIG. 7] A diagram showing effect of agitation power on yield during solvent extraction of vanillin from fermentation broth using ethyl acetate.

Modes for Carrying out the Invention

Hereinafter, the present invention will be explained in detail.
Examples of the method of the present invention include the following 1st and 2nd embodiments.
The 1st embodiment of the method of the present invention is a method comprising the following steps (1A) and (1B):

(1A) a step of treating a fermentation broth containing an objective substance with a protease; and
(1B) a step of extracting the objective substance with an organic solvent from the fermentation broth after the treatment.

In the 1st embodiment of the method of the present invention, the step (1A) is also referred to as "protease treatment" or "protease treatment step". In the 1st embodiment of the method of the present invention, the step (1B) is also referred to as "solvent extraction" or "solvent extraction step".
By carrying out the protease treatment and then carrying out the solvent extraction, emulsification during the solvent extraction can be prevented, i.e. an effect of preventing emulsification during the solvent extraction is obtained. In the 1st embodiment of the method of the present invention, this effect is also referred to as "emulsification-preventing effect". That is, the method of the present invention (specifically, the 1st embodiment of the method of the present invention) may be a method for preventing emulsification upon extracting an objective substance with an organic solvent from a fermentation broth containing the objective substance.
The 2nd embodiment of the method of the present invention is a method comprising the following step (2A):

(2A) a step of extracting an objective substance with an organic solvent from a fermentation broth containing the objective substance,

wherein the step (2A) is carried out by stirring the fermentation broth and the organic solvent with an adjusted agitation power.

In the 2nd embodiment of the method of the present invention, the step (2A) is also referred to as "solvent extraction" or "solvent extraction step".

By carrying out the solvent extraction with an adjusted agitation power, emulsification during the solvent extraction can be prevented, i.e. an effect of preventing emulsification during the solvent extraction is obtained. In the 2nd embodiment of the method of the present invention, this effect is also referred to as "emulsification-preventing effect". That is, the method of the present invention (specifically, the 2nd embodiment of the method of the present invention) may be a method for preventing emulsification upon extracting an objective substance with an organic solvent from a fermentation broth containing the objective substance.

In addition, an organic layer containing the objective substance can be efficiently separated from an aqueous layer due to the emulsification-preventing effect, and thereby the objective substance can be collected from the fermentation broth. In other words, an organic layer containing the objective substance can be efficiently separated from an aqueous layer due to the emulsification-preventing effect, and thereby the objective substance can be purified. That is, the method of the present invention may also be a method of purifying the objective substance. Also, in other words, an organic layer containing the objective substance can be efficiently separated from an aqueous layer due to the emulsification-preventing effect, and thereby the objective substance can be obtained. That is, the method of the present invention may also be a method of producing the objective substance.

The emulsification-preventing effect can be measured on the basis of the degree of emulsification during the solvent extraction as an indicator. The degree of emulsification can be measured, for example, as the ratio of the height of the emulsion layer to the height of the organic layer (OL) containing the emulsion layer [%/OL]. This ratio is also referred to as "emulsion generation amount". In the 1st embodiment of the method of the present invention, the emulsion generation amount observed when the protease treatment is carried out and then the solvent extraction is carried out may be, for example, 20[%/OL] or less, 15[%/OL] or less, 10[%/OL] or less, 5[%/OL] or less, 2[%/OL] or less, 1[%/OL] or less, or zero. The emulsion generation amount observed when the protease treatment is carried out and then the solvent extraction is carried out may be, for example, 0.2 times or less, 0.15 times or less, 0.1 times or less, 0.05 times or less, 0.02 times or less, 0.01 times or less, or zero, of the emulsion generation amount observed when the solvent extraction is carried out without the protease treatment. In the 2nd embodiment of the method of the present invention, the emulsion generation amount during the solvent extraction may be, for example, 20[%/OL] or less, 15[%/OL] or less, 10[%/OL] or less, 5[%/OL] or less, 2[%/OL] or less, 1 [%/OL] or less, or zero. The degree of emulsification may mean the degree of emulsification 5 minutes after stopping the operation of the solvent extraction (i.e. in the case of the 1st embodiment of the method of the present invention, for example, stirring or shaking; in the case of the 2nd embodiment of the method of the present invention, stirring), unless otherwise stated.

<1> Fermentation broth containing objective substance and production of the same

The term "fermentation broth containing an objective substance" refers to a liquid material containing an objective substance and obtained by using a microorganism having an objective substance-producing ability (i.e. by the action of a microorganism having an objective substance-producing ability). The fermentation broth containing the objective substance is also referred to as "fermentation broth of the objective substance" or simply as "fermentation broth".

The objective substance is not particularly limited, so long as it can be extracted with an organic solvent from the fermentation broth. Examples of the objective substance can include aldehydes. Examples of the aldehydes can include aromatic aldehydes. Examples of the aromatic aldehydes can include vanillin, benzaldehyde, and cinnamaldehyde. Particular examples of the aromatic aldehydes can include vanillin. The fermentation broth may contain one kind of objective substance, or two or more kinds of objective substances.

The fermentation broth containing the objective substance can be obtained by using a microorganism having an objective substance-producing ability (i.e. by the action of a microorganism having an objective substance-producing ability). Specifically, the objective substance can be generated by using a microorganism having an objective substance-producing ability (i.e. by the action of a microorganism having an objective substance-producing ability), and thereby the fermentation broth containing the objective substance can be obtained. The method of the present invention may comprise a step of obtaining the fermentation broth in such a manner.

The objective substance may be generated, for example, from a carbon source or from a precursor of the objective substance. The case where the objective substance is generated from a carbon source is also referred to as "fermentation in a narrow sense". The case where the objective substance is generated from a precursor thereof is also referred to as "bioconversion". Although the term "fermentation broth" is used for convenience of explanation, the term "fermentation broth" is not limited to that obtained by fermentation in the narrow sense, but also includes that obtained by bioconversion.

<1-1> Microorganism having objective substance-producing ability

For production of the fermentation broth containing the objective substance, a microorganism having an objective substance-producing ability is used. The term "objective substance-producing ability" refers to an ability to produce the objective substance. That is, the term "microorganism having an objective substance-producing ability" can refer to a microorganism that is able to produce the objective substance.

The term "microorganism having an objective substance-producing ability" can refer to a microorganism that is able to produce the objective substance by fermentation in the narrow sense, if the microorganism is used in the fermentation in the narrow sense. That is, the term "microorganism having an objective substance-producing ability" may refer to a microorganism that is able to produce the objective substance from a carbon source. Specifically, the term "microorganism having an objective substance-producing ability" may refer to a microorganism that is able to, when being cultured in a culture medium, such as a culture medium containing a carbon source, produce and accumulate the objective substance in the culture medium to such a degree that the objective substance can be collected therefrom.

Also, the term "microorganism having an objective substance-producing ability" can refer to a microorganism that is able to produce the objective substance by bioconversion, if the microorganism is used in the bioconversion. That is, the term "microorganism having an objective substance-producing ability" may refer to a microorganism that is able to produce the objective substance from a precursor of the objective substance. Specifically, the term "microorganism having an objective substance-producing ability" may refer to a microorganism that is able to, when being cultured in a culture medium containing a carbon source and a precursor of the objective substance, produce and accumulate the objective substance in the culture medium in such a degree that the objective substance can be collected therefrom. Also, specifically, the term "microorganism having an objective substance-producing ability" may refer to a microorganism that is able to, when being allowed to act on a precursor of the objective substance in a reaction mixture, produce and accumulate the objective substance in the reaction mixture to such a degree that the objective substance can be collected from the reaction mixture.

The microorganism having an objective substance-producing ability may be able to accumulate the objective substance in the culture medium or reaction mixture in an amount of, for example, 0.01 g/L or more, 0.05 g/L or more, 0.1 g/L or more, 0.5 g/L or more, or 1.0 g/L or more.

The microorganism may be able to produce one kind of objective substance, or may be able to produce two or more kinds of objective substances. Also, the microorganism may be able to produce the objective substance from one kind of precursor of the objective substance or from two or more kinds of precursors of the objective substance.

The microorganism may inherently have an objective substance-producing ability, or may be modified so that it has an objective substance-producing ability. The microorganism having an objective substance-producing ability can be obtained by imparting an objective substance-producing ability to such a microorganism as described below, or enhancing an objective substance-producing ability of such a microorganism as described below.

The microorganism used as a parent strain for constructing the microorganism having an objective substance-producing ability is not particularly limited. Examples of the microorganism can include bacteria and yeast.

Examples of the bacteria can include bacteria belonging to the family *Enterobacteriaceae* and coryneform bacteria.

Examples of bacteria belonging to the family *Enterobacteriaceae* can include bacteria belonging to the genus *Escherichia, Enterobacter, Pantoea, Klebsiella, Serratia, Erwinia, Photorhabdus, Providencia, Salmonella, Morganella,* or the like. Specifically, bacteria classified into the family *Enterobacteriaceae* according to the taxonomy used in the NCBI (National Center for Biotechnology Information) database (www.ncbi.nlm.nih.gov/Taxonomy/Browser/wwwtax.cgi?id=91347) can be used.

The *Escherichia* bacteria are not particularly limited, and examples thereof can include those classified into the genus *Escherichia* according to the taxonomy known to those skilled in the field of microbiology. Examples of the *Escherichia* bacteria can include, for example, those described in the work of Neidhardt et al. (Backmann B.J., 1996, Derivations and Genotypes of some mutant derivatives of Escherichia coli K-12, pp.2460-2488, Table 1, In F.D. Neidhardt (ed.), Escherichia coli and Salmonella Cellular and Molecular Biology/Second Edition, American Society for Microbiology Press, Washington, D.C.). Examples of the *Escherichia* bacteria can include, for example, *Escherichia coli.* Specific examples of *Escherichia coli* can include, for example, *Escherichia coli* K-12 strains such as W3110 strain (ATCC 27325) and MG1655 strain (ATCC 47076); *Escherichia coli* K5 strain (ATCC 23506); *Escherichia coli* B strains such as BL21 (DE3) strain; and derivative strains thereof.

The *Enterobacter* bacteria are not particularly limited, and examples can include those classified into the genus *Enterobacter* according to the taxonomy known to those skilled in the field of microbiology. Examples the *Enterobacter* bacterium can include, for example, *Enterobacter agglomerans* and *Enterobacter aerogenes.* Specific examples of *Enterobacter agglomerans* can include, for example, the *Enterobacter agglomerans* ATCC 12287 strain. Specific examples of *Enterobacter aerogenes* can include, for example, the *Enterobacter aerogenes* ATCC 13048 strain, NBRC 12010 strain (Biotechnol. Bioeng., 2007, Mar. 27;98(2):340-348), and AJ110637 strain (FERM BP-10955). Examples the *Enterobacter* bacteria can also include, for example, the strains described in European Patent Application Laid-open (EP-A) No. 0952221. In addition, *Enterobacter agglomerans* can also include some strains classified as *Pantoea agglomerans.*

The *Pantoea* bacteria are not particularly limited, and examples can include those classified into the genus *Pantoea* according to the taxonomy known to those skilled in the field of microbiology. Examples the *Pantoea* bacteria can include, for example, *Pantoea ananatis, Pantoea stewartii, Pantoea agglomerans,* and *Pantoea citrea.* Specific examples of *Pantoea ananatis* can include, for example, the *Pantoea ananatis* LMG20103 strain, AJ13355 strain (FERM BP-6614), AJ13356 strain (FERM BP-6615), AJ13601 strain (FERM BP-7207), SC17 strain (FERM BP-11091), SC17(0) strain (VKPM B-9246), and SC17sucA strain (FERM BP-8646). Some of *Enterobacter* bacteria and *Erwinia* bacteria were reclassified into the genus *Pantoea* (Int. J. Syst. Bacteriol., 39, 337-345 (1989); Int. J. Syst. Bacteriol., 43, 162-173 (1993)). For example, some strains of *Enterobacter agglomerans* were recently reclassified into *Pantoea agglomerans, Pantoea ananatis, Pantoea stewartii,* or the like on the basis of nucleotide sequence analysis of 16S rRNAetc. (Int. J. Syst. Bacteriol., 39, 337-345 (1989)). The *Pantoea* bacteria can include those reclassified into the genus *Pantoea* as described above.

Examples of the *Erwinia* bacteria can include *Erwinia amylovora* and *Erwinia carotovora.* Examples of the *Klebsiella* bacteria can include *Klebsiella planticola.*

Examples of coryneform bacteria can include bacteria belonging to the genus *Corynebacterium, Brevibacterium, Microbacterium,* or the like.

Specific examples of such coryneform bacteria can include the following species.

*Corynebacterium acetoacidophilum*
*Corynebacterium acetoglutamicum*
*Corynebacterium alkanolyticum*
*Corynebacterium callunae*
*Corynebacterium crenatum*
*Corynebacterium glutamicum*
*Corynebacterium lilium*
*Corynebacterium melassecola*
*Corynebacterium thermoaminogenes (Corynebacterium efficiens)*
*Corynebacterium herculis*
*Brevibacterium divaricatum (Corynebacterium glutamicum)*
*Brevibacterium flavum (Corynebacterium glutamicum)*
*Brevibacterium immariophilum*
*Brevibacterium lactofermentum (Corynebacterium glutamicum)*
*Brevibacterium roseum*
*Brevibacterium saccharolyticum*
*Brevibacterium thiogenitalis*
*Corynebacterium ammoniagenes (Corynebacterium stationis)*
*Brevibacterium album*
*Brevibacterium cerinum*
*Microbacterium ammoniaphilum*

Specific examples of the coryneform bacteria can include the following strains.

*Corynebacterium acetoacidophilum* ATCC 13870
*Corynebacterium acetoglutamicum* ATCC 15806
*Corynebacterium alkanolyticum* ATCC 21511
*Corynebacterium callunae* ATCC 15991
*Corynebacterium crenatum* AS1.542
*Corynebacterium glutamicum* ATCC 13020, ATCC 13032, ATCC 13060, ATCC 13869, FERM BP-734
*Corynebacterium lilium* ATCC 15990
*Corynebacterium melassecola* ATCC 17965
*Corynebacterium efficiens (Corynebacterium thermoaminogenes)* AJ12340 (FERM BP-1539)
*Corynebacterium herculis* ATCC 13868
*Brevibacterium divaricatum (Corynebacterium glutamicum)* ATCC 14020
*Brevibacterium flavum (Corynebacterium glutamicum)* ATCC 13826, ATCC 14067, AJ12418 (FERM BP-2205)

*Brevibacterium immariophilum* ATCC 14068
*Brevibacterium lactofermentum* (*Corynebacterium glutamicum*) ATCC 13869
*Brevibacterium roseum* ATCC 13825
*Brevibacterium saccharolyticum* ATCC 14066
*Brevibacterium thiogenitalis* ATCC 19240
*Corynebacterium ammoniagenes* (*Corynebacterium stationis*) ATCC 6871, ATCC 6872
*Brevibacterium album* ATCC 15111
*Brevibacterium cerinum* ATCC 15112
*Microbacterium ammoniaphilum* ATCC 15354

The coryneform bacteria can include bacteria that had previously been classified into the genus *Brevibacterium,* but are presently united into the genus *Corynebacterium* (Int. J. Syst. Bacteriol., 41, 255 (1991)). Moreover, *Corynebacterium stationis* can include bacteria that had previously been classified as *Corynebacterium ammoniagenes,* but are presently re-classified into *Corynebacterium stationis* on the basis of nucleotide sequence analysis of 16S rRNA etc. (Int. J. Syst. Evol. Microbiol., 60, 874-879 (2010)).

The yeast may be budding yeast or may be fission yeast. The yeast may be haploid yeast or may be diploid or more polyploid yeast. Examples of the yeast can include yeast belonging to the genus *Saccharomyces* such as *Saccharomyces cerevisiae,* the genus *Pichia* (also referred to as the genus *Wickerhamomyces*) such as *Pichia ciferrii, Pichia sydowiorum,* and *Pichia pastoris,* the genus *Candida* such as *Candida utilis,* the genus *Hansenula* such as *Hansenula polymorpha,* and the genus *Schizosaccharomyces* such as *Schizosaccharomyces pombe.*

These strains are available from, for example, the American Type Culture Collection (Address: 12301 Parklawn Drive, Rockville, Maryland 20852 P.O. Box 1549, Manassas, VA20108, United States of America). That is, registration numbers are given to the respective strains, and the strains can be ordered by using these registration numbers (refer to www.atcc.org/). The registration numbers of the strains are listed in the catalogue of the American Type Culture Collection. These strains can also be obtained from, for example, the depositories at which the strains were deposited.

The method for imparting or enhancing an objective substance-producing ability is not particularly limited. As the method for imparting or enhancing an objective substance-producing ability, for example, known methods can be used. The method for imparting or enhancing an ability to produce aromatic aldehydes such as vanillin is disclosed in, for example, WO2018/079687, WO2018/079686, WO2018/079685, WO2018/079684, WO2018/079683, WO2017/073701, WO2018/079705, and WO2017/122747.

Hereafter, specific examples of the method for imparting or enhancing an objective substance-producing ability will be explained. Such modifications as exemplified below for imparting or enhancing an objective substance-producing ability may be independently used, or may be used in an appropriate combination.

An objective substance can be generated by the action of an enzyme that is involved in the biosynthesis of the objective substance. Such an enzyme can also be referred to as "objective substance biosynthesis enzyme". Therefore, the microorganism may have an objective substance biosynthesis enzyme. In other words, the microorganism may have a gene encoding an objective substance biosynthesis enzyme. Such a gene can also be referred to as "objective substance biosynthesis gene". The microorganism may inherently have an objective substance biosynthesis gene, or may be introduced with an objective substance biosynthesis gene. Methods for introducing a gene are described later.

Also, an objective substance-producing ability of a microorganism can be improved by increasing the activity of an objective substance biosynthesis enzyme. That is, examples of the method for imparting or enhancing an objective substance-producing ability can include a method of increasing the activity of an objective substance biosynthesis enzyme. That is, the microorganism may be modified so that the activity of an objective substance biosynthesis enzyme is increased. In the microorganism, the activity of one kind of objective substance biosynthesis enzyme may be increased, or the activities of two or more kinds of objective substance biosynthesis enzymes may be increased. Methods for increasing the activity of a protein (enzyme etc.) are described later. The activity of a protein (enzyme etc.) can be increased by, for example, increasing the expression of a gene encoding the protein.

Examples of the objective substance biosynthesis genes and objective substance biosynthesis enzymes include those of various organisms such as the microorganisms exemplified above. Specific examples of the objective substance biosynthesis genes and objective substance biosynthesis enzymes include those of *Enterobacteriaceae* bacteria (for example, *Escherichia* bacteria such as *E. coli*) and coryneform bacteria (for example, *Corynebacterium* bacteria such as *C. glutamicum*). Examples of the objective substance biosynthesis genes and objective substance biosynthesis enzymes also include those of organisms individually exemplified below. The nucleotide sequences of the objective substance biosynthesis genes of various organisms and the amino acid sequences of objective substance biosynthesis enzymes encoded thereby can be obtained, for example, from public databases such as NCBI or from technical documents such as patent documents. The same shall apply to genes and proteins used for imparting or enhancing an objective substance-producing ability other than the objective substance biosynthesis genes and objective substance biosynthesis enzymes.

The objective substance can be generated from, for example, a carbon source and/or a precursor of the objective substance. Hence, examples of the objective substance biosynthesis enzyme can include, for example, enzymes that catalyze the conversion from the carbon source and/or the precursor into the objective substance. For example, 3-dehydroshikimic acid, which is an intermediate of the vanillin biosynthesis pathway, can be produced via a part of the shikimate pathway, which may include steps catalyzed by 3-deoxy-D-arabino-heptulosonic acid 7-phosphate synthase (DAHP synthase), 3-dehydroquinate synthase, and 3-dehydroquinate dehydratase; 3-dehydroshikimic acid can be converted to protocatechuic acid by the action of 3-dehydroshikimate dehydratase (DHSD); protocatechuic acid can be converted to vanillic acid or protocatechualdehyde by the action of O-methyltransferase (OMT) or aromatic aldehyde oxidoreductase (also referred to as aromatic carboxylic acid reductase (ACAR)), respectively; and vanillic acid or protocatechualdehyde can be converted to vanillin by the action of ACAR or OMT, respectively. Also, benzaldehyde and cinnamaldehyde can be generated from, for example, benzoic acid and cinnamic acid, respectively, by the action of ACAR That is, specific examples of the objective substance biosynthesis enzyme can include, for example, DAHP synthase, 3-dehydroquinate synthase, 3-dehydroquinate dehydratase, DHSD, OMT, and ACAR (WO2018/079687, WO2018/079686, WO2018/079685, WO2018/079684, WO2018/079683, WO2017/073701, and WO2018/079705).

The term "3-deoxy-D-arabino-heptulosonic acid 7-phosphate synthase (DAHP synthase)" can refer to a protein that has the activity of catalyzing the reaction of converting D-erythrose 4-phosphate and phosphoenolpyruvic acid into 3-deoxy-D-arabino-heptulosonate 7-phosphate (DAHP) and phosphate (EC 2.5.1.54). This activity can also be referred to as a "DAHP synthase activity". A gene encoding a DAHP synthase can also be referred to as a "DAHP synthase gene". Examples of a DAHP synthase can include AroF, AroG, and AroH proteins, which are encoded by *aroF, aroG,* and *aroH* genes, respectively.

The term "3-dehydroquinate synthase" can refer to a protein that has the activity of catalyzing the reaction of dephosphorylating DAHP to generate 3-dehydroquinic acid (EC 4.2.3.4). This activity can also be referred to as a "3-dehydroquinate synthase activity". A gene encoding a 3-dehydroquinate synthase can also be referred to as a "3-dehydroquinate synthase gene". Examples of a 3-dehydroquinate synthase can include an AroB protein, which is encoded by an *aroB* gene.

The term "3-dehydroquinate dehydratase" can refer to a protein that has the activity of catalyzing the reaction of dehydrating 3-dehydroquinic acid to generate 3-dehydroshikimic acid (EC 4.2.1.10). This activity can also be referred to as a "3-dehydroquinate dehydratase activity". A gene encoding a 3-dehydroquinate dehydratase can also be referred to as a "3-dehydroquinate dehydratase gene". Examples of a 3-dehydroquinate dehydratase can include an AroD protein, which is encoded by an *aroD* gene.

The term "3-dehydroshikimate dehydratase (DHSD)" can refer to a protein that has the activity of catalyzing the reaction of dehydrating 3-dehydroshikimic acid to generate protocatechuic acid (EC 4.2.1.118). This activity can also be referred to as a "DHSD activity". A gene encoding a DHSD can also be referred to as a "DHSD gene". Examples of a DHSD can include an AsbF protein, which is encoded by an *asbF* gene. Specific examples of a DHSD can include those native to various organisms such as *Bacillus thuringiensis, Neurospora crassa,* and *Podospora pauciseta.*

The expression of a gene encoding an enzyme of the shikimate pathway, such as a DAHP synthase, 3-dehydroquinate synthase, and 3-dehydroquinate dehydratase, is repressed by the tyrosine repressor TyrR, which is encoded by a *tyrR* gene. Therefore, the activity of an enzyme of the shikimate pathway can also be increased by reducing the activity of the tyrosine repressor TyrR.

The term "O-methyltransferase (OMT)" can refer to a protein that has the activity of catalyzing the reaction of methylating hydroxyl group of a substance in the presence of a methyl group donor (EC 2.1.1.68 etc.). This activity can also be referred to as an "OMT activity". A gene encoding OMT can also be referred to as an "OMT gene". OMT can have a required substrate specificity depending on the specific biosynthesis pathway via which the objective substance is produced in production of the fermentation broth. For example, when the objective substance is produced via the conversion of protocatechuic acid into vanillic acid, OMT that is specific for at least protocatechuic acid can be used. Also, for example, when the objective substance is produced via the conversion of protocatechualdehyde into vanillin, OMT that is specific for at least protocatechualdehyde can be used. That is, specifically, the term "O-methyltransferase (OMT)" can refer to a protein that has the activity of catalyzing the reaction of methylating protocatechuic acid and/or protocatechualdehyde in the presence of a methyl group donor to generate vanillic acid and/or vanillin, that is, methylation of hydroxyl group at the meta-position. OMT may be specific for both protocatechuic acid and protocatechualdehyde as the substrate, but is not necessarily limited thereto. Examples of the methyl group donor can include S-adenosylmethionine (SAM).

Examples of an OMT can include OMTs native to various organisms, such as OMT native to *Homo sapiens* (Hs) (GenBank Accession No. NP_000745 and NP_009294), OMT native to *Arabidopsis thaliana* (GenBank Accession Nos. NP_200227 and NP_009294), OMT native to *Fragaria* x *ananassa* (GenBank Accession No. AAF28353), and other various OMTs native to mammals, plants, and microorganisms exemplified in WO2013/022881A1. Four kinds of transcript variants and two kinds of OMT isoforms are known for the OMT gene native to *Homo sapiens.* Examples of an OMT further can include OMTs native to *Bacteroidetes* bacteria, that is, bacteria belonging to the phylum *Bacteroidetes.*

Examples of the *Bacteroidetes* bacteria can include bacteria belonging to the genus *Niastella, Terrimonas, Chitinophaga,* or the like (International Journal of Systematic and Evolutionary Microbiology (2007), 57, 1828-1833). Examples of the *Niastella* bacteria can include *Niastella koreensis.* Examples of an OMT further can include mutant OMTs disclosed in WO2013/022881 or WO2018/079683.

The OMT activity can be measured by, for example, incubating the enzyme with a substrate, such as protocatechuic acid or protocatechualdehyde, in the presence of SAM, and measuring the enzyme- and substrate-dependent generation of the corresponding product, such as vanillic acid or vanillin (WO2013/022881A1).

The term "aromatic aldehyde oxidoreductase (aromatic carboxylic acid reductase; ACAR)" can refer to a protein that has an activity of catalyzing the reaction of reducing an aromatic carboxylic acid in the presence of an electron donor and ATP to generate the corresponding aromatic aldehyde (EC 1.2.99.6 etc.). This activity can also be referred to as an "ACAR activity". A gene encoding an ACAR can also be referred to as an "ACAR gene". ACAR can have a required substrate specificity depending on the specific biosynthesis pathway via which the objective substance is produced in production of the fermentation broth. For example, when the objective substance is produced via the conversion of vanillic acid into vanillin, ACAR that is specific for at least vanillic acid can be used. Also, for example, when the objective substance is produced via the conversion of protocatechuic acid into protocatechualdehyde, ACAR that is specific for at least protocatechuic acid can be used. That is, specifically, the term "ACAR" can also refer to a protein that has an activity of catalyzing the reaction of reducing vanillic acid and/or protocatechuic acid in the presence of an electron donor and ATP to generate vanillin and/or protocatechualdehyde. ACAR may be specific for both vanillic acid and protocatechuic acid, but is not necessarily limited thereto. Also, for example, when benzaldehyde is produced, ACAR that is specific for at least benzoic acid can be used. That is, specifically, the term "ACAR" can also refer to a protein that has an activity of catalyzing the reaction of reducing benzoic acid in the presence of an electron donor and ATP to generate benzaldehyde. Also, for example, when cinnamaldehyde is produced, ACAR that is specific for at least cinnamic acid can be used. That is, specifically, the term "ACAR" can also refer to a protein that has an activity of catalyzing the reaction of reducing cinnamic acid in the presence of an electron donor and ATP to generate cinnamaldehyde. Examples of the electron donor can include NADH and NADPH. ACAR may be, for example, one(s) that can use at least one of these electron donors.

Examples of an ACAR can include ACARs native to various organisms such as *Nocardia* sp. strain NRRL 5646, *Actinomyces* sp., *Clostridium thermoaceticum, Aspergillus niger, Corynespora melonis, Coriolus* sp., and *Neurospora* sp. (J. Biol. Chem., 2007, Vol. 282, No. 1, pp.478-485). The *Nocardia* sp. strain NRRL 5646 has been classified into *Nocardia iowensis.* Examples of an ACAR further can include ACARs native to other *Nocardia* bacteria such as *Nocardia brasiliensis and Nocardia vulneris.* Examples of an ACAR can also include ACARs native to *Gordonia* bacteria such as *Gordonia effusa, Novosphingobium* bacteria such as *Novosphingobium malaysiense,* and *Coccomyxa* microorganisms such as *Coccomyxa subellipsoidea* (WO2018/079705A1).

The ACAR activity can be measured by, for example, incubating the enzyme with a substrate, such as vanillic acid or protocatechuic acid, in the presence of ATP and NADPH, and measuring the enzyme- and substrate-dependent oxidation of NADPH (modification of the method described in J. Biol. Chem., 2007, Vol. 282, No. 1, pp.478-485).

ACAR can be made into an active enzyme by phosphopantetheinylation (J. Biol. Chem., 2007, Vol. 282, No. 1, pp.478-485). Therefore, ACAR activity can also be increased by increasing the activity of an enzyme that catalyzes phosphopantetheinylation of a protein, which can also be referred to as a "phosphopantetheinylation enzyme". That is, examples of the methods for imparting or enhancing an objective substance-producing ability can include a method of increasing the activity of a phosphopantetheinylation enzyme. That is, the microorganism can be modified so that the activity of a phosphopantetheinylation enzyme is increased. Examples of the phosphopantetheinylation enzyme can include phosphopantetheinyl transferase (PPT).

The term "phosphopantetheinyl transferase (PPT)" can refer to a protein that has an activity of catalyzing the reaction of phosphopantetheinylating ACAR in the presence of a phosphopantetheinyl group donor. This activity can also be referred to as a "PPT activity". A gene encoding a PPT can also be referred to as a "PPT gene". Examples of the phosphopantetheinyl group donor can include coenzyme A (CoA). Examples of a PPT can include an EntD protein, which is encoded by an *entD* gene. Specific examples of a PPT can include those native to various organisms such as PPT native to *Nocardia brasiliensis,* PPT native to *Nocardia farcinica* IFM10152 (J. Biol. Chem., 2007, Vol. 282, No. 1, pp.478-485), PPT native to *Corynebacterium glutamicum* (App. Env. Microbiol. 2009, Vol.75, No.9, pp.2765-2774), and EntD protein native to *E. coli.*

The PPT activity can be measured on the basis of, for example, enhancement of the ACAR activity observed when the enzyme is incubated with ACAR in the presence of CoA (J. Biol. Chem., 2007, Vol. 282, No. 1, pp.478-485).

Also, as described above, benzaldehyde and cinnamaldehyde can be generated from, for example, benzoic acid and cinnamic acid, respectively, by the action of ACAR. That is, examples of the objective substance biosynthesis enzyme can also include, for example, benzoic acid biosynthesis enzymes and cinnamic acid biosynthesis enzymes, as well as ACAR. Specifically, cinnamic acid can be generated from, for example, L-phenylalanine, by the action of phenylalanine ammonia lyase (PAL; EC 4.3.1.24). That is, examples of the cinnamic acid biosynthesis enzymes can include, for example, L-phenylalanine biosynthesis enzymes and PAL.

In addition, benzaldehyde can also be generated from, for example, L-phenylalanine (WO2017/122747). That is, examples of the objective substance biosynthesis enzyme can also include, for example, L-phenylalanine biosynthetic enzymes and enzymes that catalyze the conversion of L-phenylalanine into benzaldehyde. L-phenylalanine can be converted to phenylpyruvate, (S)-mandelate, benzoyl formate, and benzaldehyde in order by the action of amino acid deaminase (AAD; EC 1.4.3.2), 4-hydroxymandelate synthase (HMAS; EC 1.13.11.46), (S)-mandelate dehydrogenase (SMDH; EC 1.1.99.31), and Benzoylformate decarboxylase (BFDC; EC 4.1.1.7). That is, examples of the enzymes that catalyze the conversion of L-phenylalanine into benzaldehyde can include these enzymes.

Examples of the methods for imparting or enhancing an objective substance-producing ability can also include a method of increasing the activity of an uptake system of a substance other than the objective substance, such as a substance generated as an intermediate during production of the objective substance and a substance used as a precursor of the objective substance. That is, the microorganism may have been modified so that the activity of such an uptake system is increased. The term "uptake system of a substance" can refer to a protein having a function of incorporating the substance from the outside of a cell into the cell. This activity can also be referred to as an "uptake activity of a substance". A gene encoding such an uptake system can also be referred to as an "uptake system gene". Examples of such an uptake system can include a vanillic acid uptake system and a protocatechuic acid uptake system. Examples of the vanillic acid uptake system can include a VanK protein, which is encoded by a *vanK* gene (M.T. Chaudhry, et al., Microbiology, 2007, 153:857-865). Examples of the protocatechuic acid uptake system gene can include a PcaK protein, which is encoded by a *pcaK* gene (M.T. Chaudhry, et al., Microbiology, 2007, 153:857-865).

The uptake activity of a substance can be measured according to, for example, a known method (M. T. Chaudhry, et al., Microbiology, 2007. 153:857-865).

Examples of the methods for imparting or enhancing an objective substance-producing ability further can include a method of reducing the activity of an enzyme that is involved in the by-production of a substance other than the objective substance. Such a substance other than the objective substance can also be referred to as a "byproduct". Such an enzyme can also be referred to as a "byproduct generation enzyme". Examples of the byproduct generation enzyme can include, for example, enzymes that are involved in the utilization of the objective substance, and enzymes that catalyze a reaction branching away from the biosynthetic pathway of the objective substance to generate a substance other than the objective substance. The method for reducing the activity of a protein, such as an enzyme etc., will be described later. The activity of a protein, such as an enzyme etc., can be reduced by, for example, disrupting a gene that encodes the protein. For example, it has been reported that, in coryneform bacteria, vanillin is metabolized in the order of vanillin -> vanillic acid -> protocatechuic acid, and utilized (Current Microbiology, 2005, Vol. 51, pp.59-65). That is, specific examples of the byproduct generation enzyme can include an enzyme that catalyzes the conversion of vanillin into protocatechuic acid and enzymes that catalyze further metabolization of protocatechuic acid. Examples of such enzymes can include vanillate demethylase, protocatechuate 3,4-dioxygenase, and various enzymes that further decompose the reaction product of protocatechuate 3,4-dioxygenase to succinyl-CoA and acetyl-CoA (Appl. Microbiol. Biotechnol., 2012, Vol.95, p77-89). In addition, vanillin can be converted into vanillyl alcohol by the action of alcohol dehydrogenase (Kunjapur AM. et al., J. Am. Chem. Soc., 2014, Vol.136, p11644-11654.; Hansen EH. et al., App. Env. Microbiol., 2009, Vol.75, p2765-2774.). That is, specific examples of the byproduct generation enzyme can also include ADH. In addition, 3-dehydroshikimic acid, which is an intermediate of the biosynthetic pathway of vanillic acid and vanillin, can also be converted into shikimic acid by the action of shikimate dehydrogenase. That is, specific examples of the byproduct generation enzyme can also include shikimate dehydrogenase.

The term "vanillate demethylase" can refer to a protein having an activity for catalyzing the reaction of demethylating vanillic acid to generate protocatechuic acid. This activity can also be referred to as a "vanillate demethylase activity". A gene encoding vanillate demethylase can also be referred to as a "vanillate demethylase gene". Examples of a vanillate demethylase can include VanAB proteins, which are encoded by *vanAB* genes (Current Microbiology, 2005, Vol. 51, pp.59-65). The *vanA* gene and *vanB* gene encode the subunit A and subunit B of vanillate demethylase, respectively. To reduce the vanillate demethylase activity, both the *vanAB* genes may be disrupted or the like, or only one of the two may be disrupted or the like. The *vanAB* genes typically constitute the *vanABK* operon together with the *vanK* gene. Therefore, in order to reduce the vanillate demethylase activity, the *vanABK* operon may be totally disrupted or the like, for example, deleted. In such a case, the *vanK* gene may be introduced to a host again. For example, when vanillic acid present outside cells is used, and the *vanABK* operon is totally disrupted or the like, for example, deleted, it is preferable to introduce the *vanK* gene anew.

The vanillate demethylase activity can be measured by, for example, incubating the enzyme with a substrate i.e. vanillic acid, and measuring the enzyme- and substrate-dependent generation of a product, i.e. protocatechuic acid (J Bacteriol, 2001, Vol.183, p3276-3281).

The term "protocatechuate 3,4-dioxygenase" can refer to a protein having an activity for catalyzing the reaction of oxidizing protocatechuic acid to generate beta-Carboxy-cis,cis-muconic acid. This activity can also be referred to as a "protocatechuate 3,4-dioxygenase activity". A gene encoding protocatechuate 3,4-dioxygenase can also be referred to as a "protocatechuate 3,4-dioxygenase gene". Examples of a protocatechuate 3,4-dioxygenase can include PcaGH proteins, which are encoded by *pcaGH* genes (Appl. Microbiol. Biotechnol., 2012, Vol.95, p77-89). The *pcaG* gene and *pcaH* gene encode the alpha subunit and beta subunit of protocatechuate 3,4-dioxygenase, respectively. To reduce the protocatechuate 3,4-dioxygenase activity, both the *pcaGH* genes may be disrupted or the like, or only one of the two may be disrupted or the like.

The protocatechuate 3,4-dioxygenase activity can be measured by, for example, incubating the enzyme with a substrate, i.e. protocatechuic acid, and measuring the enzyme- and substrate-dependent oxygen consumption (Meth. Enz., 1970, Vol. 17A, p526-529).

The term "alcohol dehydrogenase (ADH)" can refer to a protein that has an activity for catalyzing the reaction of reducing an aldehyde in the presence of an electron donor to generate an alcohol (EC 1.1.1.1, EC 1.1.1.2, EC 1.1.1.71, etc.). This activity can also be referred to as "ADH activity". A gene encoding ADH is also referred to as "ADH gene". Examples of the aldehyde used as a substrate of ADH can include aldehydes exemplified as the objective substance, e.g. aromatic aldehydes such as vanillin, benzaldehyde, and cinnamaldehyde. That is, examples of combinations of the aldehyde and alcohol referred to in the explanation of "ADH activity" can include a combination of an aromatic aldehyde and the corresponding aromatic alcohol, such as the combination of vanillin and vanillyl alcohol, the combination of benzaldehyde and benzyl alcohol, and the combination of cinnamaldehyde and cinnamyl alcohol. ADH that uses an aromatic aldehyde, vanillin, benzaldehyde, or cinnamaldehyde as a substrate can also be referred to as "aromatic alcohol dehydrogenase", "vanillyl alcohol dehydrogenase", "benzyl alcohol dehydrogenase", or "cinnamyl alcohol dehydrogenase", respectively. Furthermore, the ADH activity wherein an aromatic aldehyde, vanillin, benzaldehyde, or cinnamaldehyde is used as a substrate can also be referred to as "aromatic alcohol dehydrogenase activity", "vanillyl alcohol dehydrogenase activity", "benzyl alcohol dehydrogenase activity", or "cinnamyl alcohol dehydrogenase activity", respectively. ADH may use one kind of aldehyde as a substrate, or may use two or more kinds of aldehydes as substrates. Examples of the electron donor can include NADH and NADPH. ADH may be, for example, one(s) that can use at least one of these electron donors.

Examples of ADH can include YqhD protein, NCgl0324 protein, NCgl0313 protein, NCgl2709 protein, NCgl0219 protein, and NCgl2382 protein, which are encoded by *yqhD* gene, *NCgl0324* gene, *NCgl0313* gene, NCgl2709 gene, *NCgl0219* gene, and *NCgl2382* gene, respectively. The *yqhD* gene and the *NCgl0324* gene each encode vanillyl alcohol dehydrogenase. The *yqhD* gene can be found in, for example, bacteria belonging to the family *Enterobacteriaceae* such as *E. coli.* The *NCgl0324* gene, *NCgl0313* gene, *NCgl2709* gene, *NCgl0219* gene, and *NCgl2382* gene can be found in, for example, coryneform bacteria such as *C. glutamicum.*

In the microorganism, the activity of one kind of ADH may be reduced, or the activities of two or more kinds of ADHs may be reduced. For example, the activity or activities of one or more kinds of ADHs, e.g. all ADHs, selected from NCgl0324 protein, NCgl2709 protein, and NCgl0313 protein may be reduced. Also, at least the activity or activities of either one or both of NCgl0324 protein and NCgl2709 protein may be reduced. That is, for example, at least the activity of NCgl0324 protein may be reduced, and the activity of NCgl2709 protein may further be reduced. Alternatively, at least the activity of NCgl2709 protein may be reduced, and the activity of NCgl0324 protein may further be reduced. Combination of ADH and the objective substance is not particularly limited, so long as a reduction in the activity of ADH in a coryneform bacterium provides an increased production of the objective substance. For example, the activity of ADH that uses at least an aldehyde to be produced as the objective substance as a substrate may be reduced. That is, for example, the activity of an aromatic alcohol dehydrogenase such as vanillyl alcohol dehydrogenase, benzyl alcohol dehydrogenase, and cinnamyl alcohol dehydrogenase may be reduced for production of an aromatic aldehyde such as vanillin, benzaldehyde, and cinnamaldehyde, respectively. For example, when vanillin is produced, the activity of YqhD protein may be reduced. Also, for example, when vanillin is produced, the activity or activities of either one or both of NCgl0324 protein and NCgl0313 protein may be reduced, or at least the activity of NCgl0324 protein may be reduced. Also, for example, when benzaldehyde is produced, the activity or activities of either one or both of NCgl0324 protein and NCgl2709 protein may be reduced. Also, for example, when cinnamaldehyde is produced, the activity or activities of either one or both of NCgl0324 protein and NCgl2709 protein may be reduced. YqhD protein may have the vanillyl alcohol dehydrogenase activity. NCgl0324 protein may have all of the vanillyl alcohol dehydrogenase activity, benzyl alcohol dehydrogenase activity, and cinnamyl alcohol dehydrogenase activity. NCgl2709 protein may have both the benzyl alcohol dehydrogenase activity and cinnamyl alcohol dehydrogenase activity.

The ADH activity can be measured by, for example, incubating the enzyme with a substrate, e.g. an aldehyde such as vanillin, in the presence of NADPH or NADH, and measuring the enzyme- and substrate-dependent oxidation of NADPH or NADH.

The term "shikimate dehydrogenase" can refer to a protein that has the activity of catalyzing the reaction of reducing 3-dehydroshikimic acid in the presence of an electron donor to generate shikimic acid (EC 1.1.1.25). This activity can also be referred to as a "shikimate dehydrogenase activity". A gene encoding a shikimate dehydrogenase can also be referred to as a "shikimate dehydrogenase gene".

Examples of the electron donor can include NADH and NADPH. Shikimate dehydrogenase may be, for example, one(s) that can use at least one of these electron donors. Examples of a shikimate dehydrogenase can include an AroE protein, which is encoded by an *aroE* gene.

The shikimate dehydrogenase activity can be measured by, for example, incubating the enzyme with a substrate, such as 3-dehydroshikimic acid, in the presence of NADPH or NADH, and measuring the enzyme- and substrate-dependent oxidation of NADPH or NADH.

Examples of the methods for imparting or enhancing an objective substance-producing ability such as vanillin-producing ability further can include a method of increasing the activity of an L-cysteine biosynthesis enzyme (WO2018/079687).

The term "L-cysteine biosynthesis enzyme" can refer to a protein that is involved in L-cysteine biosynthesis. A gene encoding the L-cysteine biosynthesis enzyme can also be referred to as an "L-cysteine biosynthesis gene". Examples of the L-cysteine biosynthesis enzyme can include proteins that are involved in sulfur utilization. Examples of the proteins that are involved in sulfur utilization can include CysIXHDNYZ proteins and an Fpr2 protein, which are encoded by *cysIXHDNYZ* genes and an *fpr2* gene, respectively. The CysIXHDNYZ proteins are involved specifically in the reduction of inorganic sulfur compounds such as sulfate and sulfite. Fpr2 protein may be involved specifically in electron transport for the reduction of sulfite. Examples of the L-cysteine biosynthesis enzyme can also include O-acetylserine (thiol)-lyase. Examples of O-acetylserine (thiol)-lyase can include a CysK protein, which is encoded by a *cysK* gene. The activity of one kind of L-cysteine biosynthesis enzyme may be increased, or the activities of two or more kinds of L-cysteine biosynthesis enzymes may be increased. For example, the activities of one or more of the CysIXHDNYZ proteins, Fpr2 protein, and CysK protein may be increased, or the activities of one or more of the CysIXHDNYZ proteins and Fpr2 protein may be increased.

The activity of an L-cysteine biosynthesis enzyme can be increased by, for example, increasing the expression of a gene encoding the L-cysteine biosynthesis enzyme, that is, an L-cysteine biosynthesis gene such as the *cysIXHDNYZ* genes, *fpr2* gene, and *cysK* gene.

The expression of an L-cysteine biosynthesis gene can be increased by, for example, modifying, such as increasing or reducing, the activity of an expression regulator of the gene. That is, the expression of an L-cysteine biosynthesis gene can be increased by, for example, increasing the activity of a positive expression regulator, such as an activator, of the gene. Also, the expression of an L-cysteine biosynthesis gene can be increased by, for example, reducing the activity of a negative expression regulator, such as a repressor, of the gene. Such a regulator can also be referred to as a "regulator protein". A gene encoding such a regulator can also be referred to as a "regulator gene".

Examples of such an activator as described above can include a CysR protein and an SsuR protein, which are encoded by a *cysR* gene and an *ssuR* gene, respectively. An increased activity of the CysR protein may result in an increased expression of one or more of the *cysIXHDNYZ* genes, *fpr2* gene, and *ssuR* gene. Also, an increased activity of the SsuR protein may result in an increased expression of gene(s) involved in utilization of organic sulfur compounds. The activity or activities of either one or both of the CysR protein and SsuR protein may be increased. For example, the activity of at least the CysR protein may be increased. The activity of such an activator can be increased by, for example, increasing the expression of a gene encoding the activator.

Examples of such a repressor as described above can include the McbR protein, which is encoded by the *mcbR* gene. A reduced activity of the McbR protein may result in an increased expression of one or more of the *cysR* gene and *ssuR* gene, and thereby may further result in an increased expression of one or more of the *cysIXHDNYZ* genes and *fpr2* gene. The activity of such a repressor can be reduced by, for example, reducing the expression of a gene encoding the repressor or by disrupting a gene encoding the repressor.

That is, specifically, the activity of an L-cysteine biosynthesis enzyme can be increased by, for example, increasing the expression of one or more of the *cysIXHDNYZ* genes, *fpr2* gene, *cysR* gene, and *ssuR* gene. Therefore, the phrase "the activity of an L-cysteine biosynthesis enzyme is increased" can mean that, for example, the expression of one or more of the *cysIXHDNYZ* genes, *fpr2* gene, *cysR* gene, and *ssuR* gene is increased. For example, the expression of at least the *cysR* gene may be increased. Also, for example, the expression of all of these genes may be increased. The expression of one or more of the *cysIXHDNYZ* genes, *fpr2* gene, and *ssuR* gene may be increased by increasing the expression of the *cysR* gene.

Examples of the methods for imparting or enhancing an objective substance-producing ability such as vanillin-producing ability further can include a method of reducing the activity of the NCgl2048 protein (WO2018/079686).

The term "NCgl2048 protein" can refer to a protein encoded by an *NCgl2048* gene. Incidentally, the term "original function" of the NCgl2048 protein regarding conservative variants can mean the function of the protein having the amino acid sequence of the *NCgl2048* protein native to the *C. glutamicum* ATCC 13869 strain, or may also mean a property that a reduction in the activity of the protein in a microorganism provides an increased production of the objective substance.

Examples of the methods for imparting or enhancing an objective substance-producing ability such as vanillin-producing ability further can include a method of reducing the activity of enolase (WO2018/079684).

The term "enolase" can refer to a protein that has the activity of catalyzing the reaction of dehydrating 2-phospho-D-glyceric acid to generate phosphoenolpyruvic acid (EC 4.2.1.11). This activity can also be referred to as an "enolase activity". Enolase can also be referred to as "phosphopyruvate hydratase". A gene encoding an enolase can also be referred to as an "enolase gene". Examples of an enolase can include an Eno protein, which is encoded by an *eno* gene.

The enolase activity can be measured by, for example, incubating the enzyme with a substrate, such as 2-phospho-D-glyceric acid, and measuring the enzyme- and substrate-dependent generation of phosphoenolpyruvic acid.

Examples of the methods for imparting or enhancing an objective substance-producing ability such as vanillin-producing ability further can include a method of increasing the activity of S-adenosyl-L-homocysteine hydrolase (WO2018/079684).

The term "S-adenosyl-L-homocysteine hydrolase" can refer to a protein that has the activity of catalyzing the reaction of hydrolyzing S-adenosyl-L-homocysteine (SAH) to generate L-homocysteine and adenosine (EC 3.3.1.1). This activity can also be referred to as an "S-adenosyl-L-homocysteine hydrolase activity". S-adenosyl-L-homocysteine hydrolase can also be referred to as "adenosylhomocysteinase". A gene encoding an S-adenosyl-L-homocysteine hydrolase can also be referred to as an "S-adenosyl-L-homocysteine hydrolase gene". Examples of an S-adenosyl-L-homocysteine hydrolase can include an SahH protein, which is encoded by an *sahH* gene. Specific examples of an S-adenosyl-L-homocysteine hydrolase can include those native to various organisms such as yeast, *Streptomyces* bacteria, and coryneform bacteria.

The S-adenosyl-L-homocysteine hydrolase activity can be measured by, for example, incubating the enzyme with a substrate, such as S-adenosyl-L-homocysteine and DTNB (5,5'-Dithiobis(2-nitrobenzoic acid), and measuring the product, such as homocysteine-dependent generation of TNB (5-Mercapto-2-nitrobenzoic acid) on the basis of absorbance at 412 nm (J Mol Microbiol Biotechnol 2008, 15: 277-286).

The protein whose activity is modified can be appropriately chosen depending on the various conditions such as the type of the objective substance, the type of biosynthesis pathway that produces the objective substance, and the types and activities of the proteins inherently possessed by the microorganism. For example, when vanillin is produced by the bioconversion from protocatechuic acid, in particular, the activity or activities of one or more of OMT, ACAR, PPT, and the protocatechuic acid uptake system may be increased. Also, for example, when vanillin is produced by the bioconversion from vanillic acid, in particular, the activity or activities of one or more of ACAR, PPT, and the vanillic acid uptake system may be increased. Also, for example, when vanillin is produced by the bioconversion from protocatechualdehyde, in particular, the activity of OMT may be increased.

The genes and proteins used for breeding a microorganism having an objective substance-producing ability may have, for example, known nucleotide sequences and amino acid sequences of the above-exemplified genes and proteins (hereafter, also referred to simply as "known nucleotide sequences and amino acid sequences"), respectively. Examples of the known nucleotide sequences and amino acid sequences can include those disclosed in WO2018/079687, WO2018/079686, WO2018/079685, WO2018/079684, WO2018/079683, WO2017/073701, WO2018/079705, or WO2017/122747. The phrase "having a (nucleotide or amino acid) sequence" may mean comprising the (nucleotide or amino acid) sequence unless otherwise stated, and may also include cases of consisting of the (nucleotide or amino acid) sequence.

The genes and proteins used for breeding a microorganism having an objective substance-producing ability may also be conservative variants of the genes and proteins having the known nucleotide sequences and amino acid sequences, respectively. The term "conservative variant" refers to a variant that maintains the original function thereof. Examples of the conservative variants can include, for example, homologues and artificially modified versions of the genes and proteins having the known nucleotide sequences and amino acid sequences.

The expression "the original function is maintained" means that a variant of gene or protein has a function (activity or property) corresponding to the function (activity or property) of the original gene or protein. The expression "the original function is maintained" in reference to a gene means that a variant of the gene encodes a protein that maintains the original function. For example, the expression "the original function is maintained" in reference to an ACAR gene means that the variant of the gene encodes ACAR. Also, the expression "the original function is maintained" in reference to ACAR means that the variant of the protein has ACAR activity. The activity of each protein can be measured, for example, by methods disclosed in WO2018/079687, WO2018/079686, WO2018/079685, WO2018/079684, WO2018/079683, WO2017/073701, WO2018/079705, or WO2017/122747.

Hereafter, examples of the conservative variants will be explained.

Homologues of the genes or proteins used for breeding a microorganism having an objective substance-producing ability can be easily obtained from public databases by, for example, BLAST search or FASTA search, using any of the known nucleotide sequences and amino acid sequences as a query sequence. Furthermore, homologues of the genes used for breeding a microorganism having an objective substance-producing ability can be obtained by, for example, PCR using a chromosome of an organism such as coryneform bacteria as the template, and oligonucleotides prepared on the basis of any of the known nucleotide sequences as primers.

The genes used for breeding a microorganism having an objective substance-producing ability each may be a gene encoding a protein having any of the known amino acid sequences, including substitution, deletion, insertion, and/or addition of one or several amino acid residues at one or several positions, so long as the original function is maintained. For example, the encoded protein may have an extended or deleted N-terminus and/or C-terminus. Although the number meant by the phrase "one or several" may differ depending on the positions of amino acid residues in the three-dimensional structure of the protein or the types of amino acid residues, it means, specifically, for example, 1 to 50, 1 to 40, 1 to 30, preferably 1 to 20, more preferably 1 to 10, still more preferably 1 to 5, or particularly preferably 1 to 3.

The aforementioned substitution, deletion, insertion, or addition of one or several amino acid residues are each a conservative mutation that maintains the normal function of the protein. Typical examples of the conservative mutation are conservative substitutions. The conservative substitution is a mutation wherein substitution takes place mutually among Phe, Trp, and Tyr, if the substitution site is an aromatic amino acid; among Leu, Ile, and Val, if it is a hydrophobic amino acid; between Gln and Asn, if it is a polar amino acid; among Lys, Arg, and His, if it is a basic amino acid; between Asp and Glu, if it is an acidic amino acid; and between Ser and Thr, if it is an amino acid having a hydroxyl group. Examples of substitutions considered as conservative substitutions can include, specifically, substitution of Ser or Thr for Ala, substitution of Gln, His, or Lys for Arg, substitution of Glu, Gln, Lys, His, or Asp for Asn, substitution of Asn, Glu, or Gln for Asp, substitution of Ser or Ala for Cys, substitution of Asn, Glu, Lys, His, Asp, or Arg for Gln, substitution of Gly, Asn, Gln, Lys, or Asp for Glu, substitution of Pro for Gly, substitution of Asn, Lys, Gln, Arg, or Tyr for His, substitution of Leu, Met, Val, or Phe for Ile, substitution of Ile, Met, Val, or Phe for Leu, substitution of Asn, Glu, Gln, His, or Arg for Lys, substitution of Ile, Leu, Val, or Phe for Met, substitution of Trp, Tyr, Met, Ile, or Leu for Phe, substitution of Thr or Ala for Ser, substitution of Ser or Ala for Thr, substitution of Phe or Tyr for Trp, substitution of His, Phe, or Trp for Tyr, and substitution of Met, Ile, or Leu for Val. Furthermore, such substitution, deletion, insertion, addition, inversion, or the like of amino acid residues as mentioned above can include a naturally occurring mutation due to an individual difference, or a difference of species of the organism from which the gene is derived (mutant or variant).

Furthermore, the genes used for breeding a microorganism having an objective substance-producing ability each may be a gene encoding a protein having an amino acid sequence having an identity of, for example, 50% or more, 65% or more, 80% or more, preferably 90% or more, more preferably 95% or more, still more preferably 97% or more, or particularly preferably 99% or more, to the total amino acid sequence of any of the known amino acid sequences, so long as the original function is maintained.

Furthermore, the genes used for breeding a microorganism having an objective substance-producing ability each may be a gene, such as a DNA, that is able to hybridize under stringent conditions with a probe that can be prepared from any of the known nucleotide sequences, such as a sequence complementary to the whole sequence or a partial sequence of any of the known nucleotide sequences, so long as the original function is maintained. The "stringent conditions" refers to conditions under which a so-called specific hybrid is formed, and a non-specific hybrid is not formed. Examples of the stringent conditions can include those under which highly identical DNAs hybridize to each other, for example, DNAs not less than 50%, 65%, 80%, preferably 90%, more preferably 95%, still more preferably 97%, or particularly preferably 99% identical, hybridize to each other, and DNAs less identical than the above do not hybridize to each other, or conditions of washing of typical Southern hybridization, that is, conditions of washing once, or 2 or 3 times, at a salt concentration and temperature corresponding to 1 x SSC, 0.1% SDS at 60°C; 0.1 x SSC, 0.1% SDS at 60°C; or 0.1 x SSC, 0.1% SDS at 68°C.

The probe used for the aforementioned hybridization may be a part of a sequence that is complementary to the gene as described above. Such a probe can be prepared by PCR using oligonucleotides prepared on the basis of any of the known nucleotide sequences as primers and a DNA fragment containing any of the aforementioned genes as a template. As the probe, for example, a DNA fragment having a length of about 300 bp can be used. When a DNA fragment having a length of about 300 bp is used as the probe, in particular, the washing conditions of the hybridization may be, for example, 50°C, 2 x SSC and 0.1% SDS.

Furthermore, since properties concerning degeneracy of codons can change depending on the host, the genes used for breeding a microorganism having an objective substance-producing ability can include substitution of respective equivalent codons for any codons.

The term "identity" between amino acid sequences means an identity between the amino acid sequences calculated by blastp with default scoring parameters (i.e. Matrix, BLOSUM62; Gap Costs, Existence = 11, Extension = 1; Compositional Adjustments, Conditional compositional score matrix adjustment). The term "identity" between nucleotide sequences means an identity between the nucleotide sequences calculated by blastn with default scoring parameters (i.e. Match/Mismatch Scores = 1, - 2; Gap Costs = Linear).

<Methods for increasing activity of protein>

Hereafter, the methods for increasing the activity of a protein will be explained.

The expression "the activity of a protein is increased" can mean that the activity of the protein is increased as compared with a non-modified strain. Specifically, the expression "the activity of a protein is increased" may mean that the activity of the protein per cell is increased as compared with that of a non-modified strain. The term "non-modified strain" referred to herein can refer to a control strain that has not been modified so that the activity of an objective protein is increased. Examples of the non-modified strain can include a wild-type strain and parent strain. Specific examples of the non-modified strain can include the respective type strains of the species of microorganisms. Specific examples of the non-modified strain can also include strains exemplified above in relation to the description of microorganisms. That is, in an embodiment, the activity of a protein may be increased as compared with a type strain, i.e. the type strain of the species to which the microorganism having an objective substance-producing ability belongs. In another embodiment, the activity of a protein may also be increased as compared with the *C. glutamicum* ATCC 13869 strain. In another embodiment, the activity of a protein may also be increased as compared with the *C. glutamicum* ATCC 13032 strain. In another embodiment, the activity of a protein may also be increased as compared with the *E. coli* K-12 MG1655 strain. The state that "the activity of a protein is increased" may also be expressed as "the activity of a protein is enhanced". More specifically, the expression "the activity of a protein is increased" may mean that the number of molecules of the protein per cell is increased, and/or the function of each molecule of the protein is increased as compared with those of a non-modified strain. That is, the term "activity" in the expression "the activity of a protein is increased" is not limited to the catalytic activity of the protein, but may also mean the transcription amount of a gene (i.e. the amount of mRNA) encoding the protein, or the translation amount of the protein (i.e. the amount of the protein). Furthermore, the state that "the activity of a protein is increased" can include not only a state that the activity of an objective protein is increased in a strain inherently having the activity of the objective protein, but also a state that the activity of an objective protein is imparted to a strain not inherently having the activity of the objective protein. Furthermore, so long as the activity of the protein is eventually increased, the activity of an objective protein inherently contained in a host may be attenuated and/or eliminated, and then an appropriate type of the objective protein may be imparted to the host.

The degree of the increase in the activity of a protein is not particularly limited, so long as the activity of the protein is increased as compared with a non-modified strain. The activity of the protein may be increased to, for example, 1.2 times or more, 1.5 times or more, 2 times or more, or 3 times or more of that of a non-modified strain. Furthermore, when the non-modified strain does not have the activity of the objective protein, it is sufficient that the protein is produced as a result of introduction of the gene encoding the protein, and for example, the protein may be produced to such an extent that the activity thereof can be measured.

The modification for increasing the activity of a protein can be attained by, for example, increasing the expression of a gene encoding the protein. The expression "the expression of a gene is increased" can mean that the expression of the gene is increased as compared with a non-modified strain such as a wild-type strain and parent strain. Specifically, the expression "the expression of a gene is increased" may mean that the expression amount of the gene per cell is increased as compared with that of a non-modified strain. More specifically, the expression "the expression of a gene is increased" may mean that the transcription amount of the gene (i.e. the amount of mRNA) is increased, and/or the translation amount of the gene (i.e. the amount of the protein expressed from the gene) is increased. The state that "the expression of a gene is increased" can also be referred to as "the expression of a gene is enhanced". The expression of a gene may be increased to, for example, 1.2 times or more, 1.5 times or more, 2 times or more, or 3 times or more of that of a non-modified strain. Furthermore, the state that "the expression of a gene is increased" can include not only a state that the expression amount of an objective gene is increased in a strain that inherently expresses the objective gene, but also a state that the gene is introduced into a strain that does not inherently express the objective gene, and expressed therein. That is, the phrase "the expression of a gene is increased" may also mean, for example, that an objective gene is introduced into a strain that does not possess the gene, and is expressed therein.

The expression of a gene can be increased by, for example, increasing the copy number of the gene.

The copy number of a gene can be increased by introducing the gene into the chromosome of a host. A gene can be introduced into a chromosome by, for example, using homologous recombination (Miller, J.H., Experiments in Molecular Genetics, 1972, Cold Spring Harbor Laboratory). Examples of the gene transfer method utilizing homologous recombination can include, for example, a method of using a linear DNA such as Red-driven integration (Datsenko, K.A., and Wanner, B.L., Proc. Natl. Acad. Sci. USA, 97:6640-6645 (2000)), a method of using a plasmid containing a temperature sensitive replication origin, a method of using a plasmid capable of conjugative transfer, a method of using a suicide vector not having a replication origin that functions in a host, and a transduction method using a phage. Only one copy, or two or more copies of a gene may be introduced. For example, by performing homologous recombination using a sequence which is present in multiple copies on a chromosome as a target, multiple copies of a gene can be introduced into the chromosome. Examples of such a sequence which is present in multiple copies on a chromosome can include repetitive DNAs, and inverted repeats located at the both ends of a transposon. Alternatively, homologous recombination may be performed by using an appropriate sequence on a chromosome such as a gene unnecessary for the production of the objective substance as a target. Furthermore, a gene can also be randomly introduced into a chromosome by using a transposon or Mini-Mu (Japanese Patent Laid-open (Kokai) No. 2-109985, U.S. Patent No. 5,882,888, EP 805867 B1).

Introduction of a target gene into a chromosome can be confirmed by Southern hybridization using a probe having a sequence complementary to the whole gene or a part thereof, PCR using primers prepared on the basis of the sequence of the gene, or the like.

Furthermore, the copy number of a gene can also be increased by introducing a vector containing the gene into a host. For example, the copy number of a target gene can be increased by ligating a DNA fragment containing the target gene with a vector that functions in a host to construct an expression vector of the gene, and transforming the host with the expression vector. The DNA fragment containing the target gene can be obtained by, for example, PCR using the genomic DNA of a microorganism having the target gene as the template. As the vector, a vector autonomously replicable in the cell of the host can be used. The vector can be a multi-copy vector. Furthermore, the vector may have a marker such as an antibiotic resistance gene for selection of transformant. Furthermore, the vector may have a promoter and/or terminator for expressing the introduced gene. The vector may be, for example, a vector derived from a bacterial plasmid, a vector derived from a yeast plasmid, a vector derived from a bacteriophage, cosmid, phagemid, or the like. Specific examples of vector autonomously replicable in *Enterobacteriaceae* bacteria such as *Escherichia coli* can include, for example, pUC19, pUC18, pHSG299, pHSG399, pHSG398, pBR322, pSTV29 (all of these are available from Takara), pACYC184, pMW219 (NIPPON GENE), pTrc99A (Pharmacia), pPROK series vectors (Clontech), pKK233-2 (Clontech), pET series vectors (Novagen), pQE series vectors (QIAGEN), pCold TF DNA (Takara Bio), pACYC series vectors, and the broad host spectrum vector RSF1010. Specific examples of vector autonomously replicable in coryneform bacteria can include, for example, pHM1519 (Agric. Biol. Chem., 48, 2901-2903 (1984)); pAM330 (Agric. Biol. Chem., 48, 2901-2903 (1984)); plasmids obtained by improving these and having a drug resistance gene; plasmid pCRY30 described in Japanese Patent Laid-open (Kokai) No. 3-210184; plasmids pCRY21, pCRY2KE, pCRY2KX, pCRY31, pCRY3KE, and pCRY3KX described in Japanese Patent Laid-open (Kokai) No. 2-72876 and U.S. Patent No. 5,185,262; plasmids pCRY2 and pCRY3 described in Japanese Patent Laid-open (Kokai) No. 1-191686; pAJ655, pAJ611, and pAJ1844 described in Japanese Patent Laid-open (Kokai) No. 58-192900; pCG1 described in Japanese Patent Laid-open (Kokai) No. 57-134500; pCG2 described in Japanese Patent Laid-open (Kokai) No. 58-35197; pCG4 and pCG11 described in Japanese Patent Laid-open (Kokai) No. 57-183799; pPK4 described in U.S. Patent No. 6,090,597; pVK4 described in Japanese Patent Laid-open (Kokai) No. 9-322774; pVK7 described in Japanese Patent Laid-open (Kokai) No. 10-215883; pVK9 described in WO2007/046389; pVS7 described in WO2013/069634; and pVC7 described in Japanese Patent Laid-open (Kokai) No. 9-070291. Specific examples of vector autonomously replicable in coryneform bacteria can also include, for example, pVC7H2, which is a variant of pVC7.

When a gene is introduced, it is sufficient that the gene is able to be expressed by a host. Specifically, it is sufficient that the gene is harbored by a host so that it is expressed under control of a promoter that functions in the host. The term "a promoter that functions in a host" can refer to a promoter that shows a promoter activity in the host. The promoter may be a promoter derived from the host, or a heterogenous promoter. The promoter may be the native promoter of the gene to be introduced, or a promoter of another gene. As the promoter, for example, such a stronger promoter as described herein may also be used.

A terminator for termination of gene transcription may be located downstream of the gene. The terminator is not particularly limited so long as it functions in a host. The terminator may be a terminator derived from the host, or a heterogenous terminator. The terminator may be the native terminator of the gene to be introduced, or a terminator of another gene. Specific examples of the terminator can include, for example, T7 terminator, T4 terminator, fd phage terminator, *tet* terminator, and *trpA* terminator.

Vectors, promoters, and terminators available in various microorganisms are disclosed in detail in "Fundamental Microbiology Vol. 8, Genetic Engineering, KYORITSU SHUPPAN CO., LTD, 1987", and those can be used.

Furthermore, when two or more of genes are introduced, it is sufficient that the genes each are harbored by a host in such a manner that the genes are able to be expressed. For example, all the genes may be carried by a single expression vector or a chromosome. Furthermore, the genes may be separately carried by two or more expression vectors, or separately carried by a single or two or more expression vectors and a chromosome. An operon made up of two or more genes may also be introduced. When "introducing two or more genes", for example, respective genes encoding two or more kinds of proteins (such as enzymes), respective genes encoding two or more subunits constituting a single protein complex (such as enzyme complex), or a combination of these genes may be introduced.

The gene to be introduced is not particularly limited so long as it encodes a protein that functions in the host. The gene to be introduced may be a gene derived from the host, or may be a heterogenous gene. The gene to be introduced can be obtained by, for example, PCR using primers designed on the basis of the nucleotide sequence of the gene, and using the genomic DNA of an organism having the gene, a plasmid carrying the gene, or the like as a template. The gene to be introduced may also be totally synthesized, for example, on the basis of the nucleotide sequence of the gene (Gene, 60(1), 115-127 (1987)). The obtained gene can be used as it is, or after being modified as required. That is, a variant of a gene may be obtained by modifying the gene. A gene can be modified by a known technique. For example, an objective mutation can be introduced into an objective site of DNA by the site-specific mutation method. That is, the coding region of a gene can be modified by the site-specific mutation method so that a specific site of the encoded protein includes substitution, deletion, insertion, and/or addition of amino acid residues. Examples of the site-specific mutation method can include the method utilizing PCR (Higuchi, R., 61, in PCR Technology, Erlich, H.A. Eds., Stockton Press (1989); Carter, P., Meth. in Enzymol., 154, 382 (1987)), and the method utilizing phage (Kramer, W. and Frits, H.J., Meth. in Enzymol., 154, 350 (1987); Kunkel, T.A. et al., Meth. in Enzymol., 154, 367 (1987)). Alternatively, a variant of a gene may be totally synthesized.

Incidentally, when a protein functions as a complex consisting of a plurality of subunits, a part or all of these subunits may be modified, so long as the activity of the protein is eventually increased. That is, for example, when the activity of a protein is increased by increasing the expression of a gene, the expression of a part or all of these genes that encode the respective subunits may be enhanced. It is usually preferable to enhance the expression of all of those genes encoding the subunits. Furthermore, the subunits constituting the complex may be derived from one kind of organism or two or more kinds of organisms, so long as the complex has a function of the objective protein. That is, for example, genes of the same organism encoding a plurality of subunits may be introduced into a host, or genes of different organisms encoding a plurality of subunits may be introduced into a host.

Furthermore, the expression of a gene can be increased by improving the transcription efficiency of the gene. In addition, the expression of a gene can also be increased by improving the translation efficiency of the gene. The transcription efficiency of the gene and the translation efficiency of the gene can be improved by, for example, modifying an expression control sequence of the gene. The term "expression control sequence" collectively can refer to sites that affect the expression of a gene. Examples of the expression control sequence can include, for example, promoter, Shine-Dalgarno (SD) sequence (also referred to as ribosome binding site (RBS)), and spacer region between RBS and the start codon. Expression control sequences can be identified by using a promoter search vector or gene analysis software such as GENETYX. These expression control sequences can be modified by, for example, a method of using a temperature sensitive vector, or the Red driven integration method (WO2005/010175).

The transcription efficiency of a gene can be improved by, for example, replacing the promoter of the gene on a chromosome with a stronger promoter. The term "stronger promoter" can mean a promoter providing an improved transcription of a gene compared with an inherently existing wild-type promoter of the gene. Examples of stronger promoters can include, for example, the known high expression promoters such as T7 promoter, *trp* promoter, *lac* promoter, *thr* promoter, *tac* promoter, *trc* promoter, *tet* promoter, *araBAD* promoter, *rpoH* promoter, *msrA* promoter, Pm1 promoter (derived from the genus *Bifidobacterium*), PR promoter, and PL promoter. Examples of stronger promoters usable in coryneform bacteria can include, for example, the artificially modified P54-6 promoter (Appl. Microbiol. Biotechnol., 53, 674-679 (2000)), *pta, aceA, aceB, adh,* and *amyE* promoters inducible in coryneform bacteria with acetic acid, ethanol, pyruvic acid, or the like, and *cspB, SOD,* and *tuf* (EF-Tu) promoters, which are potent promoters capable of providing a large expression amount in coryneform bacteria (Journal of Biotechnology, 104 (2003) 311-323; Appl. Environ. Microbiol., 2005 Dec; 71 (12):8587-96), as well as P2 promoter (WO2018/079684), P3 promoter (WO2018/079684), *lac* promoter, *tac* promoter, *trc* promoter, and F1 promoter. Furthermore, as the stronger promoter, a highly-active type of an inherent promoter may also be obtained by using various reporter genes. For example, by making the -35 and -10 regions in a promoter region closer to the consensus sequence, the activity of the promoter can be enhanced (WO00/18935). Examples of highly active-type promoter can include various tac-like promoters (Katashkina JI et al., Russian Federation Patent Application No. 2006134574). Methods for evaluating the strength of promoters and examples of strong promoters are described in the paper of Goldstein et al. (Prokaryotic Promoters in Biotechnology, Biotechnol. Annu. Rev., 1, 105-128 (1995)), and so forth.

The translation efficiency of a gene can be improved by, for example, replacing the Shine-Dalgarno (SD) sequence (also referred to as ribosome binding site (RBS)) for the gene on a chromosome with a stronger SD sequence. The "stronger SD sequence" can mean a SD sequence that provides an improved translation of mRNA compared with the inherent wild-type SD sequence of the gene. Examples of stronger SD sequences can include, for example, RBS of the gene *10* derived from phage T7 (Olins P.O. et al, Gene, 1988, 73, 227-235). Furthermore, it is known that substitution, insertion, or deletion of several nucleotides in a spacer region between RBS and the start codon, especially in a sequence immediately upstream of the start codon (5'-UTR), significantly affects the stability and translation efficiency of mRNA, and hence, the translation efficiency of a gene can also be improved by modifying them.

The translation efficiency of a gene can also be improved by, for example, modifying codons. For example, the translation efficiency of the gene can be improved by replacing a rare codon present in the gene with a synonymous codon that is more frequently used. That is, the gene to be introduced may be modified, for example, so as to contain optimal codons according to the frequencies of codons observed in a host to be used. Codons can be replaced by, for example, the site-specific mutation method for introducing an objective mutation into an objective site of DNA. Alternatively, a gene fragment in which objective codons are replaced may be totally synthesized. Frequencies of codons in various organisms are disclosed in the "Codon Usage Database" (www.kazusa.or.jp/codon; Nakamura, Y et al, Nucl. Acids Res., 28, 292 (2000)).

Furthermore, the expression of a gene can also be increased by amplifying a regulator that increases the expression of the gene, or deleting or attenuating a regulator that reduces the expression of the gene.

Such methods for increasing the gene expression as mentioned above may be used independently or in any combination.

Furthermore, the modification that increases the activity of a protein can also be attained by, for example, enhancing the specific activity of the enzyme. Enhancement of the specific activity can also include desensitization to feedback inhibition. That is, when a protein is subject to feedback inhibition by a metabolite, the activity of the protein can be increased by mutating a gene or protein in a host so as to desensitize the feedback inhibition. The expression "desensitization to feedback inhibition" can include complete elimination of the feedback inhibition, and attenuation of the feedback inhibition, unless otherwise stated. Also, the state of "being desensitized to feedback inhibition", i.e. the state that feedback inhibition is eliminated or attenuated, can also be referred to as "tolerant to feedback inhibition". A protein showing an enhanced specific activity can be obtained by, for example, searching various organisms. Furthermore, a highly-active type of an inherent protein may also be obtained by introducing a mutation into the existing protein. The mutation to be introduced may be, for example, substitution, deletion, insertion, and/or addition of one or several amino acid residues at one or several positions in the protein. The mutation can be introduced by, for example, such a site-specific mutation method as mentioned above. The mutation may also be introduced by, for example, a mutagenesis treatment. Examples of the mutagenesis treatment can include irradiation of X-ray, irradiation of ultraviolet, and a treatment with a mutation agent such as N-methyl-N'-nitro-N-nitrosoguanidine (MNNG), ethyl methanesulfonate (EMS), and methyl methanesulfonate (MMS). Furthermore, a random mutation may be induced by directly treating DNA in vitro with hydroxylamine. Enhancement of the specific activity may be independently used, or may be used in any combination with such methods for enhancing gene expression as mentioned above.

The method for the transformation is not particularly limited, and conventionally known methods can be used. There can be used, for example, a method of treating recipient cells with calcium chloride so as to increase the permeability thereof for DNA, which has been reported for the *Escherichia coli* K-12 strain (Mandel, M. and Higa, A., J. Mol. Biol., 1970, 53, 159-162), and a method of preparing competent cells from cells which are in the growth phase, followed by transformation with DNA, which has been reported for *Bacillus subtilis* (Duncan, C.H., Wilson, G.A. and Young, F.E., Gene, 1997, 1:153-167). Alternatively, there can also be used a method of making DNA-recipient cells into protoplasts or spheroplasts, which can easily take up recombinant DNA, followed by introducing a recombinant DNA into the DNA-recipient cells, which is known to be applicable to *Bacillus subtilis,* actinomycetes, and yeasts (Chang, S. and Choen, S.N., 1979, Mol. Gen. Genet., 168:111-115; Bibb, M.J., Ward, J.M. and Hopwood, O.A., 1978, Nature, 274:398-400; Hinnen, A., Hicks, J.B. and Fink, G.R., 1978, Proc. Natl. Acad. Sci. USA, 75:1929-1933). Furthermore, the electric pulse method reported for coryneform bacteria (Japanese Patent Laid-open (Kokai) No. 2-207791) can also be used.

An increase in the activity of a protein can be confirmed by measuring the activity of the protein.

An increase in the activity of a protein can also be confirmed by confirming an increase in the expression of a gene encoding the protein. An increase in the expression of a gene can be confirmed by confirming an increase in the transcription amount of the gene, or by confirming an increase in the amount of a protein expressed from the gene.

An increase of the transcription amount of a gene can be confirmed by comparing the amount of mRNA transcribed from the gene with that of a non-modified strain such as a wild-type strain or parent strain. Examples of the method for evaluating the amount of mRNA can include Northern hybridization, RT-PCR, microarray, RNA-seq, and so forth (Sambrook, J., et al., Molecular Cloning A Laboratory Manual/Third Edition, Cold Spring Harbor Laboratory Press, Cold Spring Harbor (USA), 2001). The amount of mRNA may increase to, for example, 1.2 times or more, 1.5 times or more, 2 times or more, or 3 times or more of that of a non-modified strain.

An increase in the amount of a protein can be confirmed by Western blotting using antibodies (Molecular Cloning, Cold Spring Harbor Laboratory Press, Cold Spring Harbor (USA), 2001). The amount of the protein (such as the number of molecules of the protein per cell) may increase to, for example, 1.2 times or more, 1.5 times or more, 2 times or more, or 3 times or more of that of a non-modified strain.

The aforementioned methods for increasing the activity of a protein can be applied to enhancement of the activities of any proteins and enhancement of the expression of any genes.

<Method for reducing activity of protein>

Hereafter, the methods for reducing the activity of a protein will be explained.

The expression "the activity of a protein is reduced" can mean that the activity of the protein is reduced as compared with a non-modified strain. Specifically, the expression "the activity of a protein is reduced" may mean that the activity of the protein per cell is reduced as compared with that of a non-modified strain. The term "non-modified strain" referred to herein can refer to a control strain that has not been modified so that the activity of an objective protein is reduced. Examples of the non-modified strain can include a wild-type strain and parent strain. Specific examples of the non-modified strain can include the respective type strains of the species of microorganisms. Specific examples of the non-modified strain can also include strains exemplified above in relation to the description of microorganisms. That is, in an embodiment, the activity of a protein may be reduced as compared with a type strain, i.e. the type strain of the species to which the microorganism having an objective substance-producing ability belongs. In another embodiment, the activity of a protein may also be reduced as compared with the *C. glutamicum* ATCC 13869 strain. In another embodiment, the activity of a protein may also be reduced as compared with the *C. glutamicum* ATCC 13032 strain. In another embodiment, the activity of a protein may also be reduced as compared with the *E. coli* K-12 MG1655 strain. The state that "the activity of a protein is reduced" can also include a state that the activity of the protein has completely disappeared. More specifically, the expression "the activity of a protein is reduced" may mean that the number of molecules of the protein per cell is reduced, and/or the function of each molecule of the protein is reduced as compared with those of a non-modified strain. That is, the term "activity" in the expression "the activity of a protein is reduced" is not limited to the catalytic activity of the protein, but may also mean the transcription amount of a gene (i.e. the amount of mRNA) encoding the protein or the translation amount of the protein (i.e. the amount of the protein). The state that "the number of molecules of the protein per cell is reduced" can also include a state that the protein does not exist at all. The state that "the function of each molecule of the protein is reduced" can also include a state that the function of each protein molecule has completely disappeared. The degree of the reduction in the activity of a protein is not particularly limited, so long as the activity is reduced as compared with that of a non-modified strain. The activity of a protein may be reduced to, for example, 50% or less, 20% or less, 10% or less, 5% or less, or 0% of that of a non-modified strain.

The modification for reducing the activity of a protein can be attained by, for example, reducing the expression of a gene encoding the protein. The expression "the expression of a gene is reduced" can mean that the expression of the gene is reduced as compared with a non-modified strain such as a wild-type strain and parent strain. Specifically, the expression "the expression of a gene is reduced" may mean that the expression of the gene per cell is reduced as compared with that of a non-modified strain. More specifically, the expression "the expression of a gene is reduced" may mean that the transcription amount of the gene (i.e. the amount of mRNA) is reduced, and/or the translation amount of the gene (i.e. the amount of the protein expressed from the gene) is reduced. The state that "the expression of a gene is reduced" can also include a state that the gene is not expressed at all. The state that "the expression of a gene is reduced" can also be referred to as "the expression of a gene is attenuated". The expression of a gene may be reduced to, for example, 50% or less, 20% or less, 10% or less, 5% or less, or 0% of that of a non-modified strain.

The reduction in gene expression may be due to, for example, a reduction in the transcription efficiency, a reduction in the translation efficiency, or a combination of them. The expression of a gene can be reduced by modifying an expression control sequence of the gene such as a promoter, the Shine-Dalgarno (SD) sequence (also referred to as ribosome-binding site (RBS)), and a spacer region between RBS and the start codon of the gene. When an expression control sequence is modified, one or more nucleotides, two or more nucleotides, or three or more nucleotides, of the expression control sequence are modified. For example, the transcription efficiency of a gene can be reduced by, for example, replacing the promoter of the gene on a chromosome with a weaker promoter. The term "weaker promoter" can mean a promoter providing an attenuated transcription of a gene compared with an inherent wild-type promoter of the gene. Examples of weaker promoters can include, for example, inducible promoters. Examples of weaker promoters can also include, for example, P4 promoter (WO2018/079684) and P8 promoter (WO2018/079684). That is, an inducible promoter may function as a weaker promoter under a non-induced condition, such as in the absence of the corresponding inducer. Furthermore, a part of or the entire expression control sequence may be deleted. The expression of a gene can also be reduced by, for example, manipulating a factor responsible for expression control. Examples of the factor responsible for expression control can include low molecules responsible for transcription or translation control (inducers, inhibitors, etc.), proteins responsible for transcription or translation control (transcription factors etc.), nucleic acids responsible for transcription or translation control (siRNA etc.), and so forth. Furthermore, the expression of a gene can also be reduced by, for example, introducing a mutation that reduces the expression of the gene into the coding region of the gene. For example, the expression of a gene can be reduced by replacing a codon in the coding region of the gene with a synonymous codon used less frequently in a host. Furthermore, for example, the gene expression may be reduced due to disruption of a gene as described herein.

The modification for reducing the activity of a protein can also be attained by, for example, disrupting a gene encoding the protein. The expression "a gene is disrupted" can mean that a gene is modified so that a protein that can normally function is not produced. The state that "a protein that normally functions is not produced" can include a state that the protein is not produced at all from the gene, and a state that the protein of which the function (such as activity or property) per molecule is reduced or eliminated is produced from the gene.

Disruption of a gene can be attained by, for example, deleting the gene on a chromosome. The term "deletion of a gene" can refer to deletion of a partial or entire region of the coding region of the gene. Furthermore, the entire gene including sequences upstream and downstream from the coding region of the gene on a chromosome may be deleted. The region to be deleted may be any region such as an N-terminal region (i.e. a region encoding an N-terminal region of a protein), an internal region, or a C-terminal region (i.e. a region encoding a C-terminal region of a protein), so long as the activity of the protein can be reduced. Deletion of a longer region can usually more surely inactivate the gene. The region to be deleted may be, for example, a region having a length of 10% or more, 20% or more, 30% or more, 40% or more, 50% or more, 60% or more, 70% or more, 80% or more, 90% or more, or 95% or more of the total length of the coding region of the gene. Furthermore, it is preferred that reading frames of the sequences upstream and downstream from the region to be deleted are not the same. Inconsistency of reading frames may cause a frameshift downstream of the region to be deleted.

Disruption of a gene can also be attained by, for example, introducing a mutation for an amino acid substitution (missense mutation), a stop codon (nonsense mutation), addition or deletion of one or two nucleotide residues (frame shift mutation), or the like into the coding region of the gene on a chromosome (Journal of Biological Chemistry, 272:8611-8617 (1997); Proceedings of the National Academy of Sciences, USA, 95 5511-5515 (1998); Journal of Biological Chemistry, 26 116, 20833-20839 (1991)).

Disruption of a gene can also be attained by, for example, inserting another nucleotide sequence into a coding region of the gene on a chromosome. Site of the insertion may be in any region of the gene, and insertion of a longer nucleotide sequence can usually more surely inactivate the gene. It is preferred that reading frames of the sequences upstream and downstream from the insertion site are not the same. Inconsistency of reading frames may cause a frameshift downstream of the region to be deleted. The other nucleotide sequence is not particularly limited so long as a sequence that reduces or eliminates the activity of the encoded protein is chosen, and examples thereof can include, for example, a marker gene such as antibiotic resistance genes, and a gene useful for production of the objective substance.

Particularly, disruption of a gene may be carried out so that the amino acid sequence of the encoded protein is deleted. In other words, the modification for reducing the activity of a protein can be attained by, for example, deleting the amino acid sequence of the protein, specifically, modifying a gene so as to encode a protein of which the amino acid sequence is deleted. The term "deletion of the amino acid sequence of a protein" can refer to deletion of a partial or entire region of the amino acid sequence of the protein. In addition, the term "deletion of the amino acid sequence of a protein" means that the original amino acid sequence disappears in the protein, and can also include cases where the original amino acid sequence is changed to another amino acid sequence. That is, for example, a region that was changed to another amino acid sequence by frameshift may be regarded as a deleted region. When the amino acid sequence of a protein is deleted, the total length of the protein is typically shortened, but there can also be cases where the total length of the protein is not changed or is extended. For example, by deletion of a partial or entire region of the coding region of a gene, a region encoded by the deleted region can be deleted in the encoded protein. In addition, for example, by introduction of a stop codon into the coding region of a gene, a region encoded by the downstream region of the introduction site can be deleted in the encoded protein. In addition, for example, by frameshift in the coding region of a gene, a region encoded by the frameshift region can be deleted in the encoded protein. The aforementioned descriptions concerning the position and length of the region to be deleted in deletion of a gene can be applied *mutatis mutandis* to the position and length of the region to be deleted in deletion of the amino acid sequence of a protein.

Such modification of a gene on a chromosome as described above can be attained by, for example, preparing a disruption-type gene modified so that it is unable to produce a protein that functions normally, and transforming a host with a recombinant DNA containing the disruption-type gene to cause homologous recombination between the disruption-type gene and the wild-type gene on a chromosome and thereby substitute the disruption-type gene for the wild-type gene on the chromosome. In this procedure, if a marker gene selected according to the characteristics of the host such as auxotrophy is included in the recombinant DNA, the operation becomes easier. Examples of the disruption-type gene can include a gene of which a partial or entire region of the coding region is deleted, gene including a missense mutation, gene including a nonsense mutation, gene including a frame shift mutation, and gene including insertion of a transposon or marker gene. The protein encoded by the disruption-type gene has a conformation different from that of the wild-type protein, even if it is produced, and thus the function thereof is reduced or eliminated. Such gene disruption based on gene substitution utilizing homologous recombination has already been established, and there are methods of using a linear DNA such as a method called "Red driven integration" (Datsenko, K.A, and Wanner, B.L., Proc. Natl. Acad. Sci. USA, 97:6640-6645 (2000)), and a method utilizing the Red driven integration in combination with an excision system derived from λ phage (Cho, E.H., Gumport, R.I., Gardner, J.F., J. Bacteriol., 184:5200-5203 (2002)) (refer to WO2005/010175), a method of using a plasmid having a temperature sensitive replication origin, a method of using a plasmid capable of conjugative transfer, a method of utilizing a suicide vector not having a replication origin that functions in a host (U.S. Patent No. 6,303,383, Japanese Patent Laid-open (Kokai) No. 05-007491), and so forth.

The modification for reducing activity of a protein can also be attained by, for example, a mutagenesis treatment. Examples of the mutagenesis treatment can include irradiation of X-ray or ultraviolet and treatment with a mutation agent such as N-methyl-N'-nitro-N-nitrosoguanidine (MNNG), ethyl methanesulfonate (EMS), and methyl methanesulfonate (MMS).

When a protein functions as a complex consisting of a plurality of subunits, a part or all of the plurality of subunits may be modified, so long as the activity of the protein is eventually reduced. That is, for example, a part or all of genes that encode the respective subunits may be disrupted or the like. Furthermore, when there is a plurality of isozymes of a protein, a part or all of the activities of the plurality of isozymes may be reduced, so long as the activity of the protein is eventually reduced. That is, for example, a part or all of genes that encode the respective isozymes may be disrupted or the like.

Such methods for reducing the activity of a protein as mentioned above may be used independently or in any combination.

A reduction in the activity of a protein can be confirmed by measuring the activity of the protein.

A reduction in the activity of a protein can also be confirmed by confirming a reduction in the expression of a gene encoding the protein. A reduction in the expression of a gene can be confirmed by confirming a reduction in the transcription amount of the gene or a reduction in the amount of the protein expressed from the gene.

A reduction in the transcription amount of a gene can be confirmed by comparing the amount of mRNA transcribed from the gene with that observed in a non-modified strain. Examples of the method for evaluating the amount of mRNA can include Northern hybridization, RT-PCR, microarray, RNA-seq, and so forth (Molecular Cloning, Cold Spring Harbor Laboratory Press, Cold Spring Harbor (USA), 2001). The amount of mRNA can be reduced to, for example, 50% or less, 20% or less, 10% or less, 5% or less, or 0%, of that observed in a non-modified strain.

A reduction in the amount of a protein can be confirmed by Western blotting using antibodies (Molecular Cloning, Cold Spring Harbor Laboratory Press, Cold Spring Harbor (USA) 2001). The amount of the protein (such as the number of molecules of the protein per cell) can be reduced to, for example, 50% or less, 20% or less, 10% or less, 5% or less, or 0%, of that observed in a non-modified strain.

Disruption of a gene can be confirmed by determining nucleotide sequence of a part or the whole of the gene, restriction enzyme map, full length, or the like of the gene depending on the means used for the disruption.

Such methods for reducing the activity of a protein as mentioned above can be applied to reduction in the activities of any proteins and reduction in the expression of any genes.

<1-2> Production of fermentation broth containing objective substance

<1-2-1> Fermentation in the narrow sense

The fermentation broth can be produced by, for example, the fermentation in the narrow sense using the microorganism having an objective substance-producing ability. That is, the objective substance can be produced by the fermentation in the narrow sense, and thereby the fermentation broth containing the objective substance can be obtained. The step of producing the fermentation broth by the fermentation in the narrow sense is also referred to as "fermentation step".

The fermentation step can be performed by cultivating the microorganism having an objective substance-producing ability. Specifically, in the fermentation step, the objective substance can be generated from a carbon source. That is, the fermentation step may be, for example, a step of cultivating the microorganism in a culture medium, such as a culture medium containing a carbon source, to produce and accumulate the objective substance in the culture medium. Also, in other words, the fermentation step may be, for example, a step of generating the objective substance from a carbon source by using the microorganism.

The culture medium is not particularly limited, so long as the microorganism can proliferate in it and produce the objective substance. As the culture medium, for example, a culture medium that is typically used for culture of microorganisms such as bacteria and yeast can be used. The culture medium may contain a carbon source, nitrogen source, phosphate source, and sulfur source, as well as other medium components such as various organic components and inorganic components as required. The types and concentrations of the medium components can be appropriately determined according to various conditions such as the type of the microorganism to be used.

The carbon source is not particularly limited, so long as the microorganism can utilize it and produce the objective substance. Specific examples of the carbon source can include, for example, saccharides such as glucose, fructose, sucrose, lactose, galactose, xylose, arabinose, blackstrap molasses, hydrolysates of starches, and hydrolysates of biomass; organic acids such as acetic acid, citric acid, succinic acid, and gluconic acid; alcohols such as ethanol, glycerol, and crude glycerol; and fatty acids. As the carbon source, in particular, plant-derived materials can be used. Examples of the plant can include, for example, corn, rice, wheat, soybean, sugarcane, beet, and cotton. Examples of the plant-derived materials can include, for example, organs such as root, stem, trunk, branch, leaf, flower, and seed, plant bodies including them, and decomposition products of these plant organs. The forms of the plant-derived materials at the time of use thereof are not particularly limited, and they can be in any form such as an unprocessed product, juice, ground product, and purified product. Pentoses such as xylose, hexoses such as glucose, or mixtures of them can be obtained and used from, for example, plant biomass. Specifically, these saccharides can be obtained by subjecting a plant biomass to such a treatment as steam treatment, hydrolysis with concentrated acid, hydrolysis with diluted acid, hydrolysis with an enzyme such as cellulase, and alkaline treatment. Since hemicellulose is generally more easily hydrolyzed compared with cellulose, hemicellulose in a plant biomass may be hydrolyzed beforehand to liberate pentoses, and then cellulose may be hydrolyzed to generate hexoses. Furthermore, xylose may be supplied by conversion from hexoses by, for example, imparting a pathway for converting hexose such as glucose to xylose to the microorganism. As the carbon source, one kind of carbon source may be used, or two or more kinds of carbon sources may be used in combination.

The concentration of the carbon source in the culture medium is not particularly limited, so long as the microorganism can proliferate and produce the objective substance. The concentration of the carbon source in the culture medium may be as high as possible within such a range that production of the objective substance is not inhibited. Initial concentration of the carbon source in the culture medium may be, for example, 5 to 30% (w/v), or 10 to 20% (w/v). Furthermore, the carbon source may be added to the culture medium as required. For example, the carbon source may be added to the culture medium in proportion to decrease or depletion of the carbon source accompanying progress of the fermentation. While the carbon source may be temporarily depleted so long as the objective substance can be eventually produced, it may be preferable to perform the culture so that the carbon source is not depleted or the carbon source does not continue to be depleted.

Specific examples of the nitrogen source can include, for example, ammonium salts such as ammonium sulfate, ammonium chloride, and ammonium phosphate, organic nitrogen sources such as peptone, yeast extract, meat extract, and soybean protein decomposition products, ammonia, and urea. Ammonia gas and aqueous ammonia used for pH adjustment may also be used as a nitrogen source. As the nitrogen source, one kind of nitrogen source may be used, or two or more kinds of nitrogen sources may be used in combination.

Specific examples of the phosphate source can include, for example, phosphate salts such as potassium dihydrogenphosphate and dipotassium hydrogenphosphate, and phosphoric acid polymers such as pyrophosphoric acid. As the phosphate source, one kind of phosphate source may be used, or two or more kinds of phosphate sources may be used in combination.

Specific examples of the sulfur source can include, for example, inorganic sulfur compounds such as sulfates, thiosulfates, and sulfites, and sulfur-containing amino acids such as cysteine, cystine, and glutathione. As the sulfur source, one kind of sulfur source may be used, or two or more kinds of sulfur sources may be used in combination.

Specific examples of other various organic and inorganic components can include, for example, inorganic salts such as sodium chloride and potassium chloride; trace metals such as iron, manganese, magnesium, and calcium; vitamins such as vitamin B1, vitamin B2, vitamin B6, nicotinic acid, nicotinamide, and vitamin B12; amino acids; nucleic acids; and organic components containing these such as peptone, casamino acid, yeast extract, and soybean protein decomposition product. As the other various organic and inorganic components, one kind of component may be used, or two or more kinds of components may be used in combination.

Furthermore, when an auxotrophic mutant strain that requires a nutrient such as amino acids for growth thereof is used, the culture medium may preferably contain such a required nutrient. Furthermore, the culture medium may also contain a component used for production of the objective substance. Specific examples of such a component can include, for example, methyl group donors such as SAM and precursors thereof such as methionine.

Culture conditions are not particularly limited, so long as the microorganism can proliferate, and the objective substance is produced. The culture can be performed with, for example, conditions typically used for the culture of microorganisms such as bacteria and yeast. The culture conditions may be appropriately determined according to various conditions such as the type of chosen microorganism.

The culture can be performed by using a liquid medium. At the time of the culture, for example, the microorganism cultured on a solid medium such as agar medium may be directly inoculated into a liquid medium, or the microorganism cultured in a liquid medium as seed culture may be inoculated into a liquid medium for main culture. That is, the culture may be performed separately as seed culture and main culture. In such a case, the culture conditions of the seed culture and the main culture may be or may not be the same. It is sufficient that the objective substance is produced at least during the main culture. The amount of the microorganism contained in the culture medium at the time of the start of the culture is not particularly limited. For example, a seed culture broth having an OD660 of 4 to 100 may be inoculated to a culture medium for main culture in an amount of 0.1 to 100 mass %, or 1 to 50 mass %, at the time of the start of the culture.

The culture can be performed as batch culture, fed-batch culture, continuous culture, or a combination of these. The culture medium used at the start of the culture can also be referred to as "starting medium". The culture medium added to the culture system (e.g. fermentation tank) in the fed-batch culture or the continuous culture can also be referred to as "feed medium". To add a feed medium to the culture system in the fed-batch culture or the continuous culture can also be referred to as "feed". Furthermore, when the culture is performed separately as seed culture and main culture, the culture schemes of the seed culture and the main culture may be or may not be the same. For example, both the seed culture and the main culture may be performed as batch culture. Alternatively, for example, the seed culture may be performed as batch culture, and the main culture may be performed as fed-batch culture or continuous culture.

The various components such as the carbon source may be present in the starting medium, feed medium, or both. That is, the various components such as the carbon source may be added to the culture medium independently or in any combination during the culture. These components may be added once or a plurality of times, or may be continuously added. The types of the components present in the starting medium may be or may not be the same as those of the components present in the feed medium. Furthermore, the concentrations of the components present in the starting medium may be or may not be the same as the concentrations of the components present in the feed medium. Furthermore, two or more kinds of feed media having components of different types and/or different concentrations may be used. For example, when feeding is intermittently performed two or more times, the types and/or concentrations of components contained in the feed medium may be or may not be the same for each feeding.

The culture can be performed, for example, under an aerobic condition. The term "aerobic condition" may refer to a condition where the dissolved oxygen concentration in the culture medium is 0.33 ppm or higher, or 1.5 ppm or higher. The oxygen concentration can be controlled to be, for example, 1 to 50%, or about 5%, of the saturated oxygen concentration. The culture can be performed, for example, with aeration or shaking. The pH of the culture medium may be, for example, 3 to 10, or 4.0 to 9.5. The pH of the culture medium can be adjusted during the culture as required. The pH of the culture medium can be adjusted by using various alkaline and acidic substances such as ammonia gas, aqueous ammonia, sodium carbonate, sodium bicarbonate, potassium carbonate, potassium bicarbonate, magnesium carbonate, sodium hydroxide, potassium hydroxide, calcium hydroxide, and magnesium hydroxide. The culture temperature may be, for example, 20 to 45°C, or 25 to 37°C. The culture time may be, for example, 10 to 120 hours. The culture may be continued, for example, until the carbon source present in the culture medium is consumed, or until the activity of the microorganism is lost.

By cultivating the microorganism under such conditions as described above, the objective substance is accumulated in the culture medium, and thereby a fermentation broth (specifically, a culture broth) containing the objective substance is obtained.

<1-2-2> Bioconversion

The fermentation broth can also be produced by, for example, the bioconversion using the microorganism having an objective substance-producing ability. That is, the objective substance can be produced by the bioconversion, and thereby the fermentation broth containing the objective substance can be obtained. The step of producing the fermentation broth by the bioconversion is also referred to as "bioconversion step".

Specifically, in the bioconversion step, the objective substance can be produced from a precursor of the objective substance. More specifically, in the bioconversion step, the objective substance can be produced by converting a precursor of the objective substance into the objective substance by using the microorganism. That is, the bioconversion step may be a step of converting a precursor of the objective substance into the objective substance by using the microorganism.

A precursor of the objective substance can also be referred to simply as "precursor". Examples of the precursor can include intermediates of the biosynthesis pathway of the objective substance, such as those recited in relation to descriptions of the objective substance biosynthesis enzymes. The precursor may also be, for example, a substance whose conversion into the objective substance requires SAM Specific examples of the precursor can include protocatechuic acid, protocatechualdehyde, vanillic acid, benzoic acid, cinnamic acid, and L-phenylalanine. Protocatechuic acid, protocatechualdehyde, and vanillic acid each may be used as a precursor for producing, for example, vanillin. Benzoic acid and L-phenylalanine each may be used as a precursor for producing, for example, benzaldehyde. Cinnamic acid and L-phenylalanine each may be used as a precursor for producing, for example, cinnamaldehyde. As the precursor, one kind of precursor may be used, or two or more kinds of precursors may be used in combination. In cases where the precursor is a compound that can form a salt, the precursor may be used as a free compound, a salt thereof, or a mixture thereof. That is, the term "precursor" can refer to a precursor in a free form, a salt thereof, or a mixture thereof, unless otherwise stated. Examples of the salt can include, for example, sulfate salt, hydrochloride salt, carbonate salt, ammonium salt, sodium salt, and potassium salt. As the salt of the precursor, one kind of salt may be employed, or two or more kinds of salts may be employed in combination.

As the precursor, a commercial product may be used, or one appropriately prepared and obtained may be used. The method for producing the precursor is not particularly limited, and for example, known methods can be used. A precursor can be produced by, for example, a chemical synthesis method, enzymatic method, bioconversion method, fermentation method, extraction method, or a combination of these. That is, for example, the precursor of the objective substance can be produced from a further precursor thereof using an enzyme that catalyzes the conversion of such a further precursor into the precursor of the objective substance (also referred to as "precursor biosynthesis enzyme"). Furthermore, for example, the precursor of the objective substance can be produced from a carbon source or such a further precursor by using a microorganism having a precursor-producing ability. The term "microorganism having a precursor-producing ability" can refer to a microorganism that is able to generate the precursor of the objective substance from a carbon source or a further precursor thereof. For example, examples of the method for producing protocatechuic acid according to an enzymatic method or bioconversion method can include the method of converting para-cresol into protocatechuic acid using *Pseudomonas putida* KS-0180 (Japanese Patent Laid-open (Kokai) No. 7-75589), the method of converting para-hydroxybenzoic acid into protocatechuic acid using an NADH-dependent para-hydroxybenzoic acid hydroxylase (Japanese Patent Laid-open (Kokai) No. 5-244941), the method of producing protocatechuic acid by cultivating a transformant harboring a gene that is involved in the reaction of generating protocatechuic acid from terephthalic acid in a culture medium containing terephthalic acid (Japanese Patent Laid-open (Kokai) No. 2007-104942), and the method of producing protocatechuic acid from a precursor thereof by using a microorganism having protocatechuic acid-producing ability and having a reduced activity of protocatechuic acid 5-oxidase or being deficient in that activity (Japanese Patent Laid-open (Kokai) No. 2010-207094). Furthermore, examples of the method for producing protocatechuic acid by fermentation can include the method of producing protocatechuic acid by using a bacterium of the genus *Brevibacterium* and acetic acid as a carbon source (Japanese Patent Laid-open (Kokai) No. 50-89592), the method of producing protocatechuic acid by using a bacterium of the genus *Escherichia* or *Klebsiella* introduced with a gene encoding 3-dihydroshikimate dehydrogenase and glucose as a carbon source (U.S. Patent No. 5,272,073). Furthermore, vanillic acid can be produced by using protocatechuic acid as a precursor according to an enzymatic method using OMT or a bioconversion method using a microorganism having OMT (J. Am. CHm. Soc., 1998, Vol.120), or by using ferulic acid as a precursor according to a bioconversion method using *Pseudomonas* sp. AV10 (J. App. Microbiol., 2013, Vol.116, p903-910). Furthermore, protocatechualdehyde can be produced by using protocatechuic acid as a precursor according to an enzymatic method using ACAR or a bioconversion method using a microorganism having ACAR. The produced precursor can be used for the bioconversion method as it is, or after being subjected to an appropriate treatment such as concentration, dilution, drying, dissolution, fractionation, extraction, and purification, as required. That is, as the precursor, for example, a purified product purified to a desired extent may be used, or a material containing the precursor may be used. The material containing the precursor is not particularly limited so long as the microorganism can use the precursor. Specific examples of the material containing the precursor can include a culture broth obtained by cultivating a microorganism having a precursor-producing ability, a culture supernatant separated from the culture broth, and processed products thereof such as concentrated products (such as concentrated liquid) thereof and dried products thereof.

In an embodiment, the bioconversion step can be performed by, for example, cultivating the microorganism having an objective substance-producing ability. This embodiment can also be referred to as "first embodiment of the bioconversion". That is, the bioconversion step may be, for example, a step of cultivating the microorganism in a culture medium containing a precursor of the objective substance to convert the precursor into the objective substance. The bioconversion step may be, specifically, a step of cultivating the microorganism in a culture medium containing a precursor of the objective substance to produce and accumulate the objective substance in the culture medium.

The culture medium to be used is not particularly limited, so long as the culture medium contains the precursor of the objective substance, and the microorganism can proliferate in it and produce the objective substance. Culture conditions are not particularly limited, so long as the microorganism can proliferate, and the objective substance is produced. The descriptions concerning the culture mentioned for the fermentation in the narrow sense, such as those concerning the culture medium and culture conditions, can be applied *mutatis mutandis* to the culture in the first embodiment of the bioconversion, except that the culture medium contains the precursor in this first embodiment.

The precursor may be present in the culture medium over the whole period of the culture, or may be present in the culture medium during only a partial period of the culture. That is, the phrase "cultivating a microorganism in a culture medium containing a precursor" does not necessarily mean that the precursor is present in the culture medium over the whole period of the culture. For example, the precursor may be or may not be present in the culture medium from the start of the culture. When the precursor is not present in the culture medium at the time of the start of the culture, the precursor is added to the culture medium after the start of the culture. Timing of the addition can be appropriately determined according to various conditions such as the length of the culture period. For example, after the microorganism sufficiently grows, the precursor may be added to the culture medium. Furthermore, in any case, the precursor may be added to the culture medium as required. For example, the precursor may be added to the culture medium in proportion to decrease or depletion of the precursor accompanying generation of the objective substance. Methods for adding the precursor to the culture medium are not particularly limited. For example, the precursor can be added to the culture medium by feeding a feed medium containing the precursor to the culture medium. Furthermore, for example, the microorganism having an objective substance-producing ability and a microorganism having a precursor-producing ability can be co-cultured to allow the microorganism having a precursor-producing ability to produce the precursor in the culture medium, and thereby add the precursor to the culture medium. For the phrase "a component is added to a culture medium", the indicated "a component" may include a component that is generated or regenerated in the culture medium. These means for addition may be independently used, or may be used in an appropriate combination. The concentration of the precursor in the culture medium is not particularly limited so long as the microorganism can use the precursor as a raw material of the objective substance. The concentration of the precursor in the culture medium, for example, may be 0.1 g/L or higher, 1 g/L or higher, 2 g/L or higher, 5 g/L or higher, 10 g/L or higher, or 15 g/L or higher, or may be 200 g/L or lower, 100 g/L or lower, 50 g/L or lower, or 20 g/L or lower, or may be within a range defined with a combination thereof, in terms of the weight of the free compound. The precursor may be or may not be present in the culture medium at a concentration within the range exemplified above over the whole period of the culture. For example, the precursor may be present in the culture medium at a concentration within the range exemplified above at the time of the start of the culture, or it may be added to the culture medium so that a concentration within the range exemplified above is attained after the start of the culture. In cases where the culture is performed separately as seed culture and main culture, it is sufficient that the objective substance is produced at least during the main culture. Hence, it is sufficient that the precursor is present in the culture medium at least during the main culture, i.e. over the whole period of the main culture or during a partial period of the main culture, and that is, the precursor may be or may not be present in the culture medium during the seed culture. In such cases, terms regarding the culture, such as "culture period (period of culture)" and "start of culture", can be read as those regarding the main culture.

In another embodiment, the bioconversion step can also be performed by, for example, using cells of the microorganism having an objective substance-producing ability. This embodiment can also be referred to as "second embodiment of the bioconversion". That is, the bioconversion step may be, for example, a step of converting a precursor of the objective substance in a reaction mixture into the objective substance by using cells of the microorganism. The bioconversion step may be, specifically, a step of allowing cells of the microorganism to act on a precursor of the objective substance in a reaction mixture to generate and accumulate the objective substance in the reaction mixture. The bioconversion step performed by using such cells can also be referred to as "conversion reaction".

Cells of the microorganism can be obtained by cultivating the microorganism. The culture method for obtaining the cells is not particularly limited so long as the microorganism can proliferate. At the time of the culture for obtaining the cells, the precursor may be or may not be present in the culture medium. Also, at the time of the culture for obtaining the cells, the objective substance may be or may not be produced in the culture medium. The descriptions concerning the culture described for the fermentation in the narrow sense, such as those concerning the culture medium and culture conditions, can be applied *mutatis mutandis* to the culture for obtaining the cells used for the second embodiment of the bioconversion.

The cells may be used for the conversion reaction while being present in the culture broth (specifically, culture medium), or after being collected from the culture broth (specifically, culture medium). The cells may also be used for the conversion reaction after being subjected to a treatment as required. That is, examples of the cells can include a culture broth containing the cells, the cells collected from the culture broth, or a processed product thereof. In other words, examples of the cells can include cells present in a culture broth of the microorganism, cells collected from the culture broth, and cells present in a processed product thereof. Examples of the processed product can include products obtained by subjecting the cells to a treatment. Specific examples of the processed product can include products obtained by subjecting a culture broth containing the cells to a treatment, or subjecting the cells collected from the culture broth to a treatment. Cells in these forms may be independently used, or may be used in an appropriate combination.

The method for collecting the cells from the culture broth is not particularly limited, and for example, known methods can be used. Examples of such methods can include, for example, spontaneous precipitation, centrifugation, and filtration. A flocculant may also be used. These methods may be independently used, or may be used in an appropriate combination. The collected cells can be washed as required by using an appropriate medium. The collected cells can be re-suspended as required by using an appropriate medium. Examples of the medium that can be used for washing or suspending the cells can include, for example, aqueous media (aqueous solvents) such as water and aqueous buffer.

Examples of the treatment of the cells can include, for example, dilution, condensation, immobilization on a carrier such as acrylamide and carrageenan, freezing and thawing treatment, and treatment for increasing permeability of cell membranes. Permeability of cell membranes can be increased by, for example, using a surfactant or organic solvent. These treatments may be independently used, or may be used in an appropriate combination.

The cells used for the conversion reaction are not particularly limited so long as the cells have an objective substance-producing ability. It is preferred that the cells maintain the metabolic activities thereof. The expression "the cells maintain the metabolic activities thereof" may mean that the cells have an ability to utilize a carbon source to generate or regenerate a substance required for producing the objective substance. Examples of such a substance can include, for example, ATP, electron donors such as NADH and NADPH, and methyl group donors such as SAM The cells may have or may not have proliferation ability.

The conversion reaction can be carried out in an appropriate reaction mixture. Specifically, the conversion reaction can be carried out by allowing the cells and the precursor to coexist in an appropriate reaction mixture. The conversion reaction may be carried out by the batch method or may be carried out by the column method. In the case of the batch method, the conversion reaction can be carried out by, for example, mixing the cells of the microorganism and the precursor in a reaction mixture contained in a reaction vessel. The conversion reaction may be carried out statically, or may be carried out with stirring or shaking the reaction mixture. In the case of the column method, the conversion reaction can be carried out by, for example, passing a reaction mixture containing the precursor through a column filled with immobilized cells. Examples of the reaction mixture can include those based on an aqueous medium (aqueous solvent) such as water and aqueous buffer.

The reaction mixture may contain components other than the precursor as required, in addition to the precursor. Examples of the components other than the precursor can include ATP, electron donors such as NADH and NADPH, methyl group donors such as SAM, metal ions, buffering agents, surfactants, organic solvents, carbon sources, phosphate sources, and other various medium components. That is, for example, a culture medium containing the precursor may also be used as a reaction mixture. That is, the descriptions concerning the culture medium mentioned for the first embodiment of the bioconversion may also be applied *mutatis mutandis* to the reaction mixture in the second embodiment of the bioconversion. The types and concentrations of the components contained in the reaction mixture may be determined according to various conditions such as the type of the precursor to be used and the form of the cells to be used.

Conditions of the conversion reaction, such as dissolved oxygen concentration, pH of the reaction mixture, reaction temperature, reaction time, concentrations of various components, etc., are not particularly limited so long as the objective substance is generated. The conversion reaction can be performed with, for example, conditions typically used for substance conversion using microbial cells such as resting cells. The conditions of the conversion reaction may be determined according to various conditions such as the type of microorganism. The conversion reaction can be performed, for example, under an aerobic condition. The term "aerobic condition" may refer to a condition where the dissolved oxygen concentration in the reaction mixture is 0.33 ppm or higher, or 1.5 ppm or higher. The oxygen concentration can be controlled to be, for example, 1 to 50%, or about 5%, of the saturated oxygen concentration. The pH of the reaction mixture may be, for example, usually 6.0 to 10.0, or 6.5 to 9.0. The reaction temperature may be, for example, usually 15 to 50°C, 15 to 45°C, or 20 to 40°C. The reaction time may be, for example, 5 minutes to 200 hours. In the case of the column method, the loading rate of the reaction mixture may be, for example, such a rate that the reaction time falls within the range of the reaction time exemplified above. Furthermore, the conversion reaction can also be performed with, for example, a culture condition, such as conditions typically used for culture of microorganisms such as bacteria and yeast. During the conversion reaction, the cells may or may not proliferate. That is, the descriptions concerning the culture conditions described for the first embodiment of the bioconversion may also be applied *mutatis mutandis* to the conditions of the conversion reaction in the second embodiment of the bioconversion, except that the cells may or may not proliferate in this second embodiment. In such a case, the culture conditions for obtaining the cells and the conditions of the conversion reaction may be the same or different. The concentration of the precursor in the reaction mixture, for example, may be 0.1 g/L or higher, 1 g/L or higher, 2 g/L or higher, 5 g/L or higher, 10 g/L or higher, or 15 g/L or higher, or may be 200 g/L or lower, 100 g/L or lower, 50 g/L or lower, or 20 g/L or lower, or may be within a range defined with a combination thereof, in terms of the weight of the free compound. The density of the cells in the reaction mixture, for example, may be 1 or higher, or may be 300 or lower, or may be within a range defined with a combination thereof, in terms of the optical density (OD) at 600 nm.

During the conversion reaction, the cells, the precursor, and the other components may be added to the reaction mixture independently or in any combination thereof. For example, the precursor may be added to the reaction mixture in proportion to decrease or depletion of the precursor accompanying generation of the objective substance. These components may be added once or a plurality of times, or may be continuously added.

Methods for adding the various components such as the precursor to the reaction mixture are not particularly limited. These components each can be added to the reaction mixture by, for example, directly adding them to the reaction mixture. Furthermore, for example, the microorganism having an objective substance-producing ability and a microorganism having a precursor-producing ability can be co-cultured to allow the microorganism having a precursor-producing ability to produce the precursor in the reaction mixture, and thereby add the precursor to the reaction mixture. Furthermore, for example, components such as ATP, electron donors, and methyl group donors each may be generated or regenerated in the reaction mixture, may be generated or regenerated in the cells of the microorganism, or may be generated or regenerated by a coupling reaction between different cells. For example, when cells of the microorganism maintain the metabolic activities thereof, they can generate or regenerate components such as ATP, electron donors, and methyl group donors within them by using a carbon source. For example, specifically, the microorganism may have an enhanced ability for generating or regenerating SAM, and the generated or regenerated SAM by it may be used for the conversion reaction. The generation or regeneration of SAM may further be enhanced in combination with any other method for generating or regenerating SAM In addition, examples of the method for generating or regenerating ATP can include, for example, the method of supplying ATP from a carbon source by using a *Corynebacterium* bacterium (Hori, H. et al., Appl. Microbiol. Biotechnol., 48(6):693-698 (1997)), the method of regenerating ATP by using yeast cells and glucose (Yamamoto, S et al., Biosci. Biotechnol. Biochem., 69(4):784-789 (2005)), the method of regenerating ATP using phosphoenolpyruvic acid and pyruvate kinase (C. Aug'e and Ch. Gautheron, Tetrahedron Lett., 29:789-790 (1988)), and the method of regenerating ATP by using polyphosphoric acid and polyphosphate kinase (Murata, K. et al., Agric. Biol. Chem., 52(6):1471-1477 (1988)). For the phrase "a component is added to a reaction mixture", the indicated "a component" may include a component that is generated or regenerated in the reaction mixture.

Furthermore, the reaction conditions may be constant from the start to the end of the conversion reaction, or they may vary during the conversion reaction. The phrase "the reaction conditions vary during the conversion reaction" can include not only when the reaction conditions are temporally changed, but also when the reaction conditions are spatially changed. The phrase "the reaction conditions are spatially changed" can mean that, for example, when the conversion reaction is performed by the column method, the reaction conditions such as reaction temperature and cell density differ depending on position in the flow.

By carrying out the bioconversion as described above, the objective substance is accumulated in the culture medium or reaction mixture, and thereby a fermentation broth (specifically, a culture broth or reaction mixture) containing the objective substance is obtained.

Production of the objective substance can be confirmed by known methods used for detection or identification of compounds. Examples of such methods can include, for example, HPLC, UPLC, LC/MS, GC/MS, and NMR These methods may be used in an appropriate combination. These methods can also be used for determining the concentrations of various components other than the objective substance present in the culture medium or reaction mixture.

The contained amount of the objective substance in the fermentation broth is not particularly limited, so long as the objective substance can be extracted with an organic solvent. The contained amount of the objective substance in the fermentation broth, for example, may be 0.01 g/L or more, 0.05 g/L or more, 0.1 g/L or more, 0.5 g/L or more, or 1 g/L or more, or may be 100 g/L or less, 50 g/L or less, 20 g/L or less, 10 g/L or less, 5 g/L or less, 2 g/L or less, or 1 g/L or less, or may be within a range defined by a non-contradictory combination thereof. The contained amount of the objective substance in the fermentation broth may be, specifically, for example, 0.01 to 100 g/L, 0.05 to 50 g/L, or 0.1 to 20 g/L.

The fermentation broth may contain, in addition to the objective substance, component(s) other than the objective substance. A component other than the objective substance is also referred to as "impurity". Examples of the impurities include components that cause emulsification during the solvent extraction. Examples of the components that cause emulsification during the solvent extraction include proteins. The proteins may be derived from, for example, the cells, the culture medium, the reaction mixture, or a combination thereof. The proteins may be derived from, for example, in particular, the cells. Examples of the proteins derived from the cells include proteins released from the cells during production of the fermentation broth and proteins released from the cells during a pre-treatment of the fermentation broth.

The fermentation broth (e.g. the culture broth or reaction mixture) may be subject to the subsequent step (i.e. protease treatment in the case of the 1st embodiment of the method of the present invention; solvent extraction in the case of the 2nd embodiment of the method of the present invention) as it is, or after being subject to a pre-treatment. That is, the 1st embodiment of the method of the present invention may comprise a step of subjecting the fermentation broth to a pre-treatment prior to the protease treatment. Also, the 2nd embodiment of the method of the present invention may comprise a step of subjecting the fermentation broth to a pre-treatment prior to the solvent extraction. The pre-treatment is not particularly limited, so long as the emulsification-preventing effect is not spoiled. Examples of the pre-treatment include concentration, dilution, heating, cell removal, and pH adjustment. These pretreatments may be used independently or in any appropriate combination. That is, examples of the fermentation broth to be subject to the protease treatment in the 1st embodiment of the method of the present invention or the fermentation broth to be subject to the solvent extraction in the 2nd embodiment of the method of the present invention include the fermentation broth itself obtained by using the microorganism having an objective substance-producing ability (e.g. the culture broth or reaction mixture itself obtained by using the microorganism having an objective substance-producing ability) and processed products thereof. Examples of the processed products include those that have been subject to such a pre-treatment as described above (e.g. concentration, dilution, heating, cell removal, pH adjustment, or a combination thereof). That is, specific examples of the processed products include the fermentation broth after concentration, dilution, heating, cell removal, pH adjustment, or a combination thereof. Particular examples of the processed products include the fermentation broth after cell removal. The term "fermentation broth after a certain pre-treatment" refers to a fermentation broth that has been subject at least to the certain pre-treatment, and include a fermentation broth that has been subject to other pre-treatment(s) in addition to the certain pre-treatment. For example, the term "fermentation broth after cell removal" refers to a fermentation broth that has been subject at least to cell removal, and include a fermentation broth that has been subject to other pre-treatment(s) in addition to cell removal. The fermentation broth after cell removal is also referred to as "supernatant of the fermentation broth" or simply as "supernatant". The term "cell removal" refers to removal of cells. Cell removal can be carried out, for example, by spontaneous sedimentation, centrifugation, or filtration. The remaining amount of cells in the fermentation broth after cell removal may be, for example, 10,000 cells/mL or less, 1,000 cells/mL or less, 100 cells/mL or less, 10 cells/mL or less, or zero. Alternatively, the fermentation broth may be subject to the subsequent step while containing cells. In particular, in the 2nd embodiment of the method of the present invention, the fermentation broth may be subject to the solvent extraction while containing cells. That is, the fermentation broth subject to the solvent extraction include the fermentation broth containing cells. Examples of the fermentation broth containing cells include the fermentation broth that has not been subject to cell removal. Particular examples of the fermentation broth containing cells include the fermentation broth that has not been subject to centrifugation or filtration. The fermentation broth containing cells may have been subject to pre-treatment(s) other than cell removal. The contained amount of cells in the fermentation broth containing cells, for example, may be 10,000 cells/mL or more, 100,000 cells/mL or more, 1,000,000 cells/mL or more, 10,000,000 cells/mL or more, or 100,000,000 cells/mL or more, or may be 1,000,000,000,000 cells/mL or less, or may be within a range defined by a combination thereof. Examples of pH adjustment include lowering the pH. Lowering the pH may result in, for example, sterilization of cells in the fermentation broth. The pH can be lowered by, for example, addition of an acid such as sulfuric acid. The pH after adjustment may be, for example, 2.5 to 4. Examples of heating include heating at 80 to 130°C for 1 to 60 minutes. Heating may result in, for example, sterilization of cells in the fermentation broth. Heating may also result in, for example, aggregation of cells in the fermentation broth. When the pre-treatment(s) such as concentration has been carried out, the contained amount exemplified above of each component such as the objective substance and cells in the fermentation broth represents the contained amount of each component in the fermentation broth after the pre-treatment(s).

<2> Method of the present invention

<2-1> 1st Embodiment of the method of the present invention

<Step (1A)>

The step (1A) is a step of treating the fermentation broth containing the objective substance with a protease.
The step (1A) can be carried out by bringing the fermentation broth and the protease into contact with each other. In other words, the step (1A) can be carried out by allowing the fermentation broth and the protease to coexist. The step (1A), for example, may be carried out by a batch method or may be carried out by a column method. In the case of the batch method, the step (1A) can be carried out by, for example, mixing the fermentation broth and the protease in a reaction vessel. The step (1A), for example, may be carried out under static conditions, or may be carried out under stirring or shaking conditions. For the stirring conditions, for example, the stirring conditions in the step (1B) may be applied *mutatis mutandis.* The protease may be used in any form that can act on the fermentation broth. For example, the protease in a liquid form may be mixed with the fermentation broth, or the protease in a solid form (e.g. powder or granules) may be mixed with the fermentation broth. The protease may be supplied to the reaction system only once, or may be supplied to the reaction system twice or more times. For example, the protease may be additionally supplied to the reaction system when the protease is inactivated. In the case of the column method, the step (1A) can be carried out by, for example, passing the fermentation broth through a column filled with the protease (e.g. a column on which the protease is immobilized).
The conditions of the protease treatment (e.g. type of protease, use amount of protease, treatment time, treatment temperature, and treatment pH) are not particularly limited, so long as the emulsification-preventing effect is obtained. The protease treatment may be carried out, for example, so that protein(s) in the fermentation broth are degraded. That is, for example, the emulsification-preventing effect may be obtained by degradation of protein(s) in the fermentation broth. Specific examples of the proteins degraded by the protease treatment include proteins having a molecular weight of 15 kDa or less. More specific examples of the proteins degraded by the protease treatment include proteins having a molecular weight of 10 to 15 kDa.
The term "protease" refers to an enzyme that has the activity of catalyzing the reaction of hydrolyzing a peptide bond of a protein. This activity is also referred to as "protease activity". A protease is also referred to as "proteinase". The term "protease" also include those called "peptidase".
The protease is selected from proteases derived from *Bacillus* bacteria and proteases derived from *Aspergillus* fungi. The protease is not particularly limited, so long as it is derived from a *Bacillus* bacterium or an *Aspergillus* fungus and has an emulsification-preventing ability. The term "emulsification-preventing ability" refers to a function of achieving the emulsification-preventing effect, i.e. a function that use of the protease for treating the fermentation broth prior to the solvent extraction enables preventing emulsification during the solvent extraction. Whether or not a protease has the emulsification-preventing ability can be confirmed by measuring the emulsification-preventing effect using the protease. Examples of the *Bacillus* bacteria include *Bacillus amyloliquefaciens, Bacillus cereus, Bacillus clausii, Bacillus intermedius, Bacillus lentus, Bacillus licheniformis, Bacillus stearothermophilus, Bacillus subtilis,* and *Bacillus thermoproteolyticus.* Particular examples of the *Bacillus* bacteria include *Bacillus amyloliquefaciens* and *Bacillus licheniformis.* Examples of the *Aspergillus* fungi include *Aspergillus fumigatus, Aspergillus melleus, Aspergillus niger, Aspergillus oryzae,* and *Aspergillus sojae.* Particular examples of the *Aspergillus* fungi include *Aspergillus oryzae.* The protease may be selected from, particularly, proteases derived from *Bacillus* bacteria. The protease may be, for example, an acid protease, a neutral protease, or an alkaline protease. The protease may be, particularly, a neutral protease or an alkaline protease. The protease may be, for example, an aspartate protease, a serine protease, or a metalloprotease. Examples of the proteases derived from *Bacillus* bacteria include serine proteases such as subtilisin (EC 3.4.21.62), metalloproteases such as thermolysin (EC 3.4.24.27) and bacillolysin (EC 3.4.24.28), and other various kinds of acid, neutral, or alkaline proteases. Particular examples of the proteases derived from *Bacillus* bacteria include serine proteases and metalloproteases. More particular examples of the proteases derived from *Bacillus* bacteria include subtilisin and bacillolysin. Examples of the proteases derived from *Aspergillus* fungi include neutral proteases such as NPI or NPII, alkaline proteases such as ALP, and aspartate proteases (acid proteases) such as PEPO.
As the protease, a commercially available product may be used, or one produced and obtained as required may be used.
The protease can be produced by, for example, culturing a microorganism that produces the protease. The microorganism that produces the protease may be one that inherently produces the protease, or may be one that has been modified so as to produce the protease. The microorganism that produces the protease can be obtained by, for example, introducing a gene encoding the protease into a microorganism in such a manner that the gene is able to be expressed. Culture conditions of the microorganism that produces the protease are not particularly limited so long as the microorganism can grow and produce the protease. The microorganism that produces the protease can be cultured under ordinary conditions for culturing microorganisms such as bacteria and fungi. The protease may be purified to a desired degree. The protease may contain component(s) other than the protease. Examples of the components other than the protease include enzymes other than the protease.

Examples of the commercially available product of the protease include commercially available protease preparations.

Specific examples of the commercially available proteases derived from *Bacillus* bacteria include the followings.

As neutral proteases:

Protease N "Amano" G (derived from *Bacillus subtilis,* Amano Enzyme)
Protin SD-NY10 (derived from *Bacillus amyloliquefaciens,* Amano Enzyme)
Thermoase PC10F (derived from *Bacillus stearothermophilus,* Amano Enzyme)
Brewers protease (derived from *Bacillus amyloliquefaciens,* D.S.M. Japan)
Accelerzyme NP50.000 (derived from *Bacillus amyloliquefaciens,* D.S.M. Japan)
Neutrase (derived from *Bacillus amyloliquefaciens,* Novozymes Japan)
Nucleicin (derived from *Bacillus subtilis,* H.B.I.)
Orientase 90N (derived from *Bacillus subtilis,* H.B.I.)
Cololase N (derived from *Bacillus subtilis,* Higuchi)
Aroase NS (derived from *Bacillus subtilis,* Yakult Pharmaceutical Industry)
Aroase AP-10 (derived from *Bacillus subtilis,* Yakult Pharmaceutical Industry)
Aroase NP-10 (derived from *Bacillus subtilis,* Yakult Pharmaceutical Industry)

As alkaline proteases:

Protin SD-AY10 (derived from *Bacillus licheniformis,* Amano Enzyme) Delvolase (derived from *Bacillus licheniformis,* D.S.M. Japan)
Esperase (derived from *Bacillus* sp., Novozymes Japan)
Savinase (derived from *Bacillus* sp., Novozymes Japan)
Everlase (derived from *Bacillus* sp., Novozymes Japan)
Alcalase 2.4L FG (derived from *Bacillus licheniformis,* Novozymes Japan)
Bioprase OP (derived from *Bacillus* sp., Nagase Chemtex)
Bioprase SP-20FG (derived from *Bacillus* sp., Nagase Chemtex)
Orientase 22BF (derived from *Bacillus subtilis,* H.B.I.).

Particular examples of the commercially available proteases derived from *Bacillus* bacteria include the followings.

Protin SD-NY10 (derived from *Bacillus amyloliquefaciens*)
Protin SD-AY10 (derived from *Bacillus licheniformis*)
Alcalase 2.4L FG (derived from *Bacillus licheniformis*)

Protin SD-NY10 is a protease preparation containing bacillolysin. Protin SD-AY10 and Alcalase 2.4L FG each are a protease preparation containing subtilisin.

Specific examples of the commercially available proteases derived from an *Aspergillus* fungi include the followings.

As acid proteases:

Sumizyme AP (derived from *Aspergillus niger,* Shinnihon Chemicals)
Denapsin 2P (derived from *Aspergillus* sp., Nagase Chemtex)
Orientase AY (derived from *Aspergillus niger,* H.B.I.)
Tetrase S (derived from *Aspergillus niger,* H.B.I.)
Brewers Clarex (derived from *Aspergillus niger,* D.S.M. Japan)
Varidase AFP (derived from *Aspergillus niger,* D.S.M. Japan)
Protease YP-SS (derived from *Aspergillus niger,* Yakult Pharmaceutical Industry).

As neutral proteases:

ProteAX (derived from *Aspergillus oryzae,* Amano Enzyme)
Sumizyme ACP-G (derived from *Aspergillus oryzae,* Shinnihon Chemicals)
Sumizyme LP (derived from *Aspergillus oryzae,* Shinnihon Chemicals)
Sumizyme FP-G (derived from *Aspergillus oryzae,* Shinnihon Chemicals)
Varidase FP60 (derived from *Aspergillus oryzae,* D.S.M. Japan)
Denazyme AP (derived from *Aspergillus* sp., Nagase Chemtex)
Orientase OP (derived from *Aspergillus oryzae,* H.B.I.)
Pantidase P (derived from *Aspergillus* sp., Yakult Pharmaceutical Industry)
Pantidase NP-2 (derived from *Aspergillus oryzae,* Yakult Pharmaceutical industry)

As alkaline protease:
Sumizyme MP (derived from *Aspergillus* sp., Shinnihon Chemicals).

Particular examples of the commercially available proteases derived from an *Aspergillus* fungi include the followings.

ProteAX (derived from *Aspergillus oryzae*)

Incidentally, the followings may be excluded from the protease. That is, the protease may be selected from proteases other than the followings.

Protease M "Amano" SD (derived from *Aspergillus* sp., Amano Enzyme)
Protease A "Amano" SD (derived from *Aspergillus* sp., Amano Enzyme)
Protease P "Amano" 3SD (derived from *Aspergillus* sp., Amano Enzyme).

The proteases specified with the aforementioned respective product names are not limited to those sold with those product names at the time of filing of this application, but may also include equivalent products thereof. For example, even if the product names are changed due to product renewal or the like, equivalent products after change of the product names may be included in the proteases specified with the respective product names exemplified above.

As the protease, homologues of such known proteases as exemplified above may also be used. Such homologues are not particularly limited, so long as they are those found in a *Bacillus* bacterium or *Aspergillus* fungus and having the emulsification-preventing ability. As the protease, artificially modified enzymes of such known proteases as exemplified above or homologues thereof may also be used. Such artificially modified enzymes are not particularly limited, so long as they have the emulsification-preventing ability. That is, the expression that the phrase "a protease is derived from a *Bacillus* bacterium or *Aspergillus* fungus" may also include cases where the protease is an artificially modified enzyme of a protease found in a *Bacillus* bacterium or *Aspergillus* fungus. For the homologues and artificially modified enzymes of proteases, the aforementioned descriptions concerning the variants of the genes and proteins used for breeding a microorganism having an objective substance-producing ability can be applied *mutatis mutandis.*

As the protease, one kind of protease may be used, or two or more kinds of proteases may be used in combination.

The protease activity can be measured by, for example, the Folin method. When the protease activity is measured by the Folin method, the amount of enzyme that provides an increase in a coloring substance for the Folin test regent corresponding to 1 µg of tyrosine per 1 minute at 37°C at pH8.0 when the enzymatic reaction is carried out using casein as the substrate is defined as 1 U (unit).

Specifically, measurement of the protease activity by the Folin method can be carried out by, for example, the following procedure. A protease is dissolved in a calcium acetate and sodium chloride regent solution (prepared by mixing 5 ml of a 0.2 mol/L calcium acetate solution and 2.5 ml of a 2 mol/L sodium chloride solution, and diluting the mixture to a volume of 500 ml with distilled water) with stirring, and the solution is diluted 7000 times to obtain an enzyme solution. A 160 ml aliquot of 0.05 mol/L disodium hydrogenphosphate regent solution is added to 1.2 g of casein (produced from milk), and casein is dissolved by warming on a water bath. The solution is cooled with running water, then adjusted to pH 8.0 with a sodium hydroxide regent solution, and diluted with distilled water to a volume of 200 ml to obtain a substrate solution. A 5 ml aliquot of the substrate solution is put into a test tube, and warmed at 37°C for 10 minutes, then 1 ml of the enzyme solution is added, and they are mixed. The mixture is left at 37°C for 10 minutes, then 5 ml of a trichloroacetic acid regent solution (prepared by dissolving 36 g of trichloroacetic acid and 36 g of anhydrous sodium acetate in 1.6 L of distilled water, mixing the resulting solution with 110 ml of a 6 mol/L acetic acid regent solution, and diluting the resulting mixture to a volume of2 L with distilled water) was added, they are mixed by shaking, and the resulting mixture is left again at 37°C for 30 minutes, and filtered. Then, 5 ml of a 0.55 mol/L sodium carbonate regent solution is put into a test tube, 2 ml of the filtrate and the Folin test solution (Folin-Ciocalteu's Phenol Reagent, Wako Pure Chemical Industries) diluted 3 times with distilled water are added, they are mixed, and the resulting mixture is left at 37°C for 30 minutes. Then, absorbance of the mixture is measured at a wavelength of 660 nm as absorbance of the enzymatic reaction solution using distilled water as a control. Separately, 1 ml of the enzyme solution and 5 ml of the trichloroacetic acid regent solution are mixed, then 5 ml of the substrate solution is added to the mixture, the resulting mixture is left at 37°C for 30 minutes, and blank absorbance is obtained thereafter by similar procedure. Change of the amount per unit reaction time is calculated from the value obtained by subtracting the blank absorbance from the absorbance of the enzymatic reaction solution, and the protease activity is calculated.

The protease activity can also be measured by, for example, the LNA method. When the protease activity is measured by the LNA method, the amount of enzyme that generates 1 µmol of p-nitroaniline per 1 minute at 37°C at pH7.0 when the enzymatic reaction is carried out using L-leucyl-p-nitroanilide hydrochloride as the substrate is defined as 1 U (unit).

The protease activity can be measured by, for example, the Anson method. When the protease activity is measured by the Anson method, the amount of enzyme that releases 1 µmol of tyrosine per 1 minute at 37°C at pH7.5 when the enzymatic reaction is carried out using hemoglobin as the substrate is defined as 1 Anson U (Anson unit).

The use amount of the protease, for example, may be 10 U or more, 100 U or more, 500 U or more, 1,000 U or more, 2,000 U or more, 5,000 U or more, or 10,000 U or more, or may be 500,000 U or less, 200,000 U or less, 100,000 U or less, 50,000 U or less, 20,000 U or less, 10,000 U or less, 5,000 U or less, 2,000 U or less, or 1,000 U or less, or may be within a range defined by a non-contradictory combination thereof, to 100 mL of the fermentation broth in terms of the protease activity measured by the Folin method. The use amount of the protease may be, specifically, for example, 10 to 500,000 U, 100 U to 200,000 U, or 500 U to 100,000 U to 100 mL of the fermentation broth in terms of the protease activity measured by the Folin method.

The use amount of the protease, for example, may be 0.2 U or more, 2 U or more, 5 U or more, 10 U or more, 20 U or more, 50 U or more, 100 U or more, or 200 U or more, or may be 10,000 U or less, 5,000 U or less, 2,000 U or less, 1,000 U or less, 500 U or less, 200 U or less, 100 U or less, 500 U or less, or 200 U or less, or may be within a range defined by a non-contradictory combination thereof, to 100 mL of the fermentation broth in terms of the protease activity measured by the LNA method. The use amount of the protease may be, specifically, for example, 0.2 to 10,000 U, 2 U to 5,000 U, or 10 U to 2,000 U to 100 mL of the fermentation broth in terms of the protease activity measured by the LNA method.

The use amount of the protease, for example, may be 0.5 mAU or more, 5 mAU or more, 10 mAU or more, 20 mAU or more, 50 mAU or more, 100 mAU or more, 200 mAU or more, 200 mAU or more, or 500 mAU or more, or may be 20,000 mAU or less, 10,000 mAU or less, 5,000 mAU or less, 2,000 mAU or less, 1,000 mAU or less, 500 mAU or less, 200 mAU or less, 100 mAU or less, or 500 mAU or less, or may be within a range defined by a non-contradictory combination thereof, to 100 mL of the fermentation broth in terms of the protease activity measured by the Anson method. The use amount of the protease may be, specifically, for example, 0.5 to 20,000 mAU, 5 mAU to 10,000 mAU, or 20 mAU to 5,000 mAU to 100 mL of the fermentation broth in terms of the protease activity measured by the Anson method.

The treatment time, for example, may be 5 minutes or more, 10 minutes or more, 15 minutes or more, 20 minutes or more, 30 minutes or more, 40 minutes or more, 50 minutes or more, 60 minutes or more, or 90 minutes or more, or may be 240 minutes or less, 180 minutes or less, 120 minutes or less, 90 minutes or less, 60 minutes or less, 50 minutes or less, 40 minutes or less, 30 minutes or less, 30 minutes or less, or 20 minutes or less, or may be within a range defined by a non-contradictory combination thereof. The treatment time may be, specifically, for example, 10 to 180 minutes, 20 to 120 minutes, or 30 to 90 minutes.

The treatment temperature, for example, may be 10°C or higher, 15°C or higher, 20°C or higher, 30°C or higher, 40°C or higher, 50°C or higher, or 60°C or higher, or may be 80°C or lower, 70°C or lower, 60°C or lower, 50°C or lower, or 40°C or lower, or may be within a range defined by a non-contradictory combination thereof. The treatment temperature may be, specifically, for example, 10 to 70°C, 20 to 60°C, or 30 to 50°C. The treatment temperature may or may not be controlled. The protease treatment may be carried out, for example, at a room temperature (e.g. 25°C).

The treatment pH, for example, may be 2.5 or more, 3 or more, 4 or more, 5 or more, 6 or more, 7 or more, or 8 or more, or may be 10 or less, 9 or less, 8 or less, 7 or less, or 6 or less, or may be within a range defined by a non-contradictory combination thereof. The treatment pH may be, specifically, for example, 2.5 to 10. The treatment pH may or may not be controlled.

<Step (1B)>

The step (1B) is a step of extracting the objective substance with an organic solvent from the fermentation broth after the protease treatment (i.e. the step (1A)).

That is, the step (1B) is carried out after the step (1A). That is, the term "fermentation broth" referred to in the step (1B) refers to the fermentation broth after carrying out the step (1A).

The step (1B) can be carried out by bringing the fermentation broth and the organic solvent into contact with each other. In other words, the step (1B) can be carried out by allowing the fermentation broth and the organic solvent to coexist. The step (1B) may be carried out, for example, under conditions where emulsification occurs when the fermentation broth that has not been subject to the step (1A) is used. Examples of such conditions include stirring conditions and shaking conditions. Particular examples of such conditions include stirring conditions. The phrase "emulsification occurs" may mean, for example, that the emulsion generation amount is more than 5[%/OL], more than 10[%/OL], more than 15[%/OL], more than 20[%/OL], 30[%/OL] or more, 50[%/OL] or more, 70[%/OL] or more, 90[%/OL] or more, or 100[%/OL].

The conditions for the solvent extraction (e.g. type of organic solvent, use amount of organic solvent, treatment time, treatment temperature, treatment pH, and agitation speed) are not particularly limited, so long as the objective substance is extracted. As the conditions for the solvent extraction, for example, normal conditions for solvent extraction for extracting compounds from an aqueous layer such as a fermentation broth may be used as they are, after being modified as required.

The organic solvent is not particularly limited, so long as the objective substance can be extracted from the fermentation broth. In other words, as the organic solvent, one(s) that can dissolve the objective substance and is separated from the aqueous layer can be selected. The organic solvent can be appropriately set depending on the various conditions such as the type of the objective substance. Examples of the organic solvent include aromatic hydrocarbons such as toluene; alkanes such as n-hexane and cyclohexane; esters such as methyl acetate, ethyl acetate, propyl acetate, isopropyl acetate, butyl acetate, isoamyl acetate; ethers such as diethyl ether and methyl tert-butyl ether (MTBE); and dichloromethane. Particular examples of the organic solvent include toluene, ethyl acetate, and butyl acetate. As the organic solvent, one kind of organic solvent may be used, or two or more kinds of organic solvents may be used in combination.

The use amount of the organic solvent, for example, may be 25 mL or more, 33 mL or more, 50 mL or more, 75 mL or more, 100 mL or more, 133 mL or more, or 200 mL or more, or may be 400 mL or less, 300 mL or less, 200 mL or less, 133 mL or less, 100 mL or less, 75 mL or less, or 50 mL or less, or may be within a range defined by a non-contradictory combination thereof, to 100 mL of the fermentation broth. The use amount of the organic solvent may be, specifically, for example, 33 to 300 mL, 50 to 200 mL, or 75 to 133 mL to 100 mL of the fermentation broth.

The extraction time, for example, may be 5 minutes or more, 10 minutes or more, 15 minutes or more, 20 minutes or more, 30 minutes or more, 40 minutes or more, 50 minutes or more, 60 minutes or more, or 90 minutes or more, or may be 240 minutes or less, 180 minutes or less, 120 minutes or less, 90 minutes or less, 60 minutes or less, 50 minutes or less, 40 minutes or less, 30 minutes or less, 30 minutes or less, or 20 minutes or less, or may be within a range defined by a non-contradictory combination thereof. The extraction time may be, specifically, for example, 10 to 180 minutes, 20 to 120 minutes, or 30 to 90 minutes.

The extraction temperature, for example, may be 5°C or more, 10°C or more, 15°C or more, 20°C or more, 30°C or more, 40°C or more, 50°C or more, or 60°C or more, or may be 80°C or less, 70°C or less, 60°C or less, 50°C or less, 40°C or less, 30°C or less, or 20°C or less, or may be within a range defined by a non-contradictory combination thereof. The extraction temperature may be, specifically, for example, 5 to 40°C, or 20 to 40°C. The extraction temperature may or may not be controlled. The solvent extraction may be carried out, for example, at a room temperature (e.g. 25°C).

The extraction pH (specifically, the pH of the fermentation broth to be subject to the solvent extraction), for example, may be 2.5 or more, 3 or more, 4 or more, 5 or more, 6 or more, 7 or more, or 8 or more, or may be 10 or less, 9 or less, 8 or less, 7 or less, or 6 or less, or may be within a range defined by a non-contradictory combination thereof. The extraction pH may be, specifically, for example, 2.5 to 10. The extraction pH may or may not be controlled.

Stirring can be carried out by rotating a stirring blade. The shape of the stirring blade is not particularly limited, so long as a desired agitation state is obtained. Examples of the stirring blade include paddle blades, turbine blades, propeller blades, anchor blades, and retreating blades. The stirring speed may be, for example, 100 to 3000 W/kL in terms of the agitation power. The agitation power can be calculated, for example, using Nagata's formula.

By carrying out the solvent extraction as described above, the objective substance can be collected from the fermentation broth. Specifically, by carrying out the solvent extraction as described above, the objective substance is moved to an organic layer, and thereby the organic layer containing the objective substance is obtained. That is, the objective substance may be obtained, for example, as a form of an organic layer containing the objective substance. In other words, the objective substance to be produced may be an organic layer containing the objective substance. The organic layer containing the objective substance can be collected as required. That is, the method of the present invention (specifically, the 1st embodiment of the method of the present invention) may comprise, after the step (1B), a step of collecting the organic layer containing the objective substance. The timing of collecting the organic layer is not particularly limited. The organic layer may be collected, for example, immediately after stopping the operation of the solvent extraction (e.g. stirring or shaking), or after a suitable time has elapsed after stopping the operation of the solvent extraction.

The objective substance may be further purified from the organic layer. That is, the method of the present invention (specifically, the 1st embodiment of the method of the present invention) may comprise a step of purifying the objective substance from the organic layer containing the objective substance. The objective substance may be purified to a desired degree. Purification of the objective substance can be carried out, for example, by known techniques for separating and purifying compounds in organic solvents. Examples of such techniques include washing, crystallization, distillation, drying, resin treatment, and membrane treatment. These techniques may be used independently or in any combination.

<2-2> 2nd Embodiment of the method of the present invention

<Step (2A)>

The step (2A) is a step of extracting the objective substance with an organic solvent from the fermentation broth.

The step (2A) can be carried out by bringing the fermentation broth and the organic solvent into contact with each other. In other words, the step (2A) can be carried out by allowing the fermentation broth and the organic solvent to coexist. When bringing the fermentation broth and the organic solvent into contact with each other, for example, the organic solvent may be gently layered onto the fermentation broth so that emulsification does not occur.

In the step (2A), the fermentation broth and the organic solvent are stirred with an adjusted agitation power. The conditions for the solvent extraction (e.g. type of organic solvent, use amount of organic solvent, treatment time, treatment temperature, treatment pH, and agitation speed) are not particularly limited, so long as the fermentation broth and the organic solvent are stirred with an adjusted agitation power and the objective substance is extracted. As the conditions for the solvent extraction, for example, normal conditions for solvent extraction for extracting compounds from an aqueous layer such as a fermentation broth may be used as they are, after being modified as required, provided that the fermentation broth and the organic solvent are stirred with an adjusted agitation power.

The organic solvent is not particularly limited, so long as the objective substance can be extracted from the fermentation broth. In other words, as the organic solvent, one(s) that can dissolve the objective substance and is separated from the aqueous layer can be selected. The organic solvent can be appropriately set depending on the various conditions such as the type of the objective substance. Examples of the organic solvent include aromatic hydrocarbons such as toluene; alkanes such as n-hexane and cyclohexane; esters such as methyl acetate, ethyl acetate, propyl acetate, isopropyl acetate, butyl acetate, isoamyl acetate; ethers such as diethyl ether and methyl tert-butyl ether (MTBE); and dichloromethane. Particular examples of the organic solvent include toluene, ethyl acetate, and butyl acetate. As the organic solvent, one kind of organic solvent may be used, or two or more kinds of organic solvents may be used in combination.

The use amount of the organic solvent, for example, may be 25 mL or more, 33 mL or more, 50 mL or more, 75 mL or more, 100 mL or more, 133 mL or more, or 200 mL or more, or may be 400 mL or less, 300 mL or less, 200 mL or less, 133 mL or less, 100 mL or less, 75 mL or less, or 50 mL or less, or may be within a range defined by a non-contradictory combination thereof, to 100 mL of the fermentation broth. The use amount of the organic solvent may be, specifically, for example, 33 to 300 mL, 50 to 200 mL, or 75 to 133 mL to 100 mL of the fermentation broth.

The extraction time, for example, may be 30 minutes or more, 1 hours or more, 2 hours or more, 3 hours or more, 4 hours or more, 5 hours or more, or 6 hours or more, or may be 120 hours or less, 96 hours or less, 72 hours or less, 48 hours or less, 36 hours or less, 24 hours or less, 21 hours or less, 18 hours or less, 15 hours or less, 12 hours or less, 9 hours or less, 6 hours or less, 5 hours or less, 4 hours or less, 3 hours or less, 2 hours or less, or 1 hours or less, or may be within a range defined by a non-contradictory combination thereof. The extraction time may be, specifically, for example, 30 minutes to 120 hours, 1 to 72 hours, or 2 to 36 hours.

The extraction temperature, for example, may be 5°C or more, 10°C or more, 15°C or more, 20°C or more, 30°C or more, 40°C or more, 50°C or more, or 60°C or more, or may be 80°C or less, 70°C or less, 60°C or less, 50°C or less, 40°C or less, 30°C or less, or 20°C or less, or may be within a range defined by a non-contradictory combination thereof. The extraction temperature may be, specifically, for example, 50 to 80°C. The extraction temperature may or may not be controlled. The solvent extraction may be carried out, for example, at a room temperature (e.g. 25°C).

The extraction pH (specifically, the pH of the fermentation broth to be subject to the solvent extraction), for example, may be 2.5 or more, 3 or more, 4 or more, 5 or more, 6 or more, 7 or more, or 8 or more, or may be 10 or less, 9 or less, 8 or less, 7 or less, or 6 or less, or may be within a range defined by a non-contradictory combination thereof. The extraction pH may be, specifically, for example, 2.5 to 10. The extraction pH may or may not be controlled.

Stirring can be carried out by rotating a stirring blade. The shape of the stirring blade is not particularly limited, so long as a desired agitation state is obtained. Examples of the stirring blade include paddle blades, turbine blades, propeller blades, anchor blades, and retreating blades.

Stirring is carried out with an adjusted agitation power.

The agitation power may be adjusted to, for example, an agitation power with which the liquid-liquid interface (i.e. the interface between the aqueous layer and the organic layer) is maintained. The phrase "the liquid-liquid interface is maintained" may mean that the difference in height of the liquid-liquid interface (i.e. the difference between the highest and lowest parts of the liquid-liquid interface) during stirring in terms of the ratio to the height of the organic layer (OL) containing the emulsion layer [%/OL] is maintained to be 20[%/OL] or less, 10[%/OL] or less, 5[%/OL] or less, 2[%/OL] or less, 1 [%/OL] or less, or zero.

The agitation power is more than 0. The agitation power, for example, may be more than 0, 0.001 W/kL or more, 0.01 W/kL or more, 0.1 W/kL or more, 0.2 W/kL or more, 0.5 W/kL or more, 0.7 W/kL or more, 1 W/kL or more, 1.2 W/kL or more, 1.5 W/kL or more, 2 W/kL or more, 3 W/kL or more, 4 W/kL or more, or 5 W/kL or more, or may be 25 W/kL or less, 20 W/kL or less, 17 W/kL or less, 15 W/kL or less, 12 W/kL or less, 10 W/kL or less, 7 W/kL or less, or 5 W/kL or less, or may be within a range defined by a non-contradictory combination thereof. The agitation power, specifically, for example, may be more than 0 and 20 W/kL or less, 0.5 to 20 W/kL, or 1.5 to 20 W/kL, may be more than 0 and 15 W/kL or less, 0.5 to 15 W/kL, or 1.5 to 15 W/kL, may be more than 0 and 10 W/kL or less, 0.5 to 10 W/kL, or 1.5 to 10 W/kL. The agitation power may be more than 0 and 20 W/kL or less, 0.5 to 20 W/kL, or 1.5 to 20 W/kL when the organic solvent is toluene. The agitation power may be more than 0 and 10 W/kL or less, 0.5 to 10 W/kL, or 1.5 to 10 W/kL when the organic solvent is ethyl acetate.

The agitation power should be calculated using the following formula (Nagata's formula).

$$P = Np \cdot \rho \cdot n^3 \cdot d^5$$

*Np:* Power number [-]
$\rho$: Liquid density [kg/m$^3$]
*n*: Rotation speed [1/s]
*d*: Diameter of blade [m]

$$N_P = \frac{A}{\mathrm{Re}} + B\left(\frac{10^3 + 1.2\,\mathrm{Re}^{0.66}}{10^3 + 3.2\,\mathrm{Re}^{0.66}}\right)^P \left(\frac{H}{D}\right)^{(0.35 + b'/D)}$$

$$A = 14 + (b'/D)\{670(d/D - 0.6)^2 + 185\}$$

$$B = 10^{(1.3 - 4(b'/D - 0.5)^2 - 1.14(d/D))}$$

$$p = 1.1 + 4(b'/D) - 2.5(d/D - 0.5)^2 - 7(b'/D)^4$$

*Re:* Reynolds number [-]
*H*: Liquid depth [m]
*D*: Diameter of tank [m]
*b*: Width of blade [m]
*b'*= (Number of blades $\times$ b)/2 (corrected for two blades)

In the step (2A), for example, stirring may be temporally stopped. That is, in the step (2A), stirring may be carried out continuously or intermittently. The length of "temporally" as for the step (2A) is not particularly limited, so long as the objective substance can be extracted. The term "temporally" as for the step (2A) may refer to, for example, a period having a length of 20% or less, 15% or less, 10% or less, 5% or less, 3% or less, or 1% or less of the whole period of the step (2A).

By carrying out the solvent extraction as described above, the objective substance can be collected from the fermentation broth. Specifically, by carrying out the solvent extraction as described above, the objective substance is moved to an organic layer, and thereby the organic layer containing the objective substance is obtained. That is, the objective substance may be obtained, for example, as a form of an organic layer containing the objective substance. In other words, the objective substance to be produced may be an organic layer containing the objective substance. The organic layer containing the objective substance can be collected as required. That is, the method of the present invention (specifically, the 2nd embodiment of the method of the present invention) may comprise, after the step (2A), a step of collecting the organic layer containing the objective substance. The timing of collecting the organic layer is not particularly limited. The organic layer may be collected, for example, immediately after stopping the operation of the solvent extraction (e.g. stirring or shaking), or after a suitable time has elapsed after stopping the operation of the solvent extraction.

The objective substance may be further purified from the organic layer. That is, the method of the present invention (specifically, the 2nd embodiment of the method of the present invention) may comprise a step of purifying the objective substance from the organic layer containing the objective substance. The objective substance may be purified to a desired degree. Purification of the objective substance can be carried out, for example, by known techniques for separating and purifying compounds in organic solvents. Examples of such techniques include washing, crystallization, distillation, drying, resin treatment, and membrane treatment. These techniques may be used independently or in any combination.

Examples

Hereafter, the present invention will be more specifically explained with reference to the following non-limiting examples.

<1> Evaluation of emulsification-preventing effect of protease (1)

In this example, a fermentation broth containing vanillin was treated with a protease and then subjected to solvent extraction using an organic solvent, to thereby evaluate the emulsification-preventing effect of the protease treatment. As the protease, Alcalase 2.4L FG (derived from *Bacillus licheniformis,* Novozymes Japan) was used. As the organic solvent, toluene was used.

*Corynebacterium glutamicum* introduced with the aromatic carboxylic acid reductase (ACAR) derived from *Gordonia effusa* (WO2018/079705) was cultured in a culture medium containing vanillic acid, to thereby obtain a culture broth containing 5.2 g/L vanillin. The culture broth was adjusted to pH3 by addition of 98% sulfuric acid and kept at 50°C for 30 minutes, to thereby obtain a sterilized broth. The sterilized broth was kept at 120°C for 3 minutes, to thereby obtain a heat-aggregated broth. The obtained heat-aggregated broth was designated as "1612BP heat-aggregated broth".

A centrifugal supernatant ($9190 \times$ g, 5 min) of the 1612BP heat-aggregated broth was weighed into screw-top bottles in 10 mL portions. The centrifugal supernatant was adjusted to pH 7.0, then added with 0 to 1 wt% of Alcalase 2.4L FG, and allowed to stand at 40°C for 1 hour. The reaction mixture was cooled to room temperature, then added with 10 mL of toluene, and stirred vigorously using a stirrer at room temperature. One hour later, stirring was stopped, the mixture was allowed to stand for about 5 minutes, and then the degree of emulsification was observed. The degree of emulsification was measured as the ratio of the height of the emulsion layer to the height of the organic layer (OL) containing the emulsion layer [%/OL]. This ratio is also referred to as "emulsion generation amount". Separately, the reaction mixture before addition of toluene was sampled and subjected to SDS-PAGE, to analyze contaminated proteins.

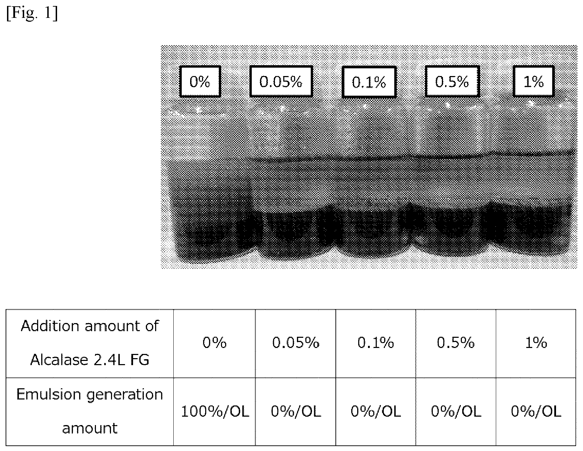

The observed results of emulsification are shown in Figure 1. Emulsification was observed throughout in the organic layer of the sample without Alcalase 2.4L FG treatment, whereas no emulsification was observed in the sample treated with Alcalase 2.4L FG. A sufficient emulsification-preventing effect was obtained even when 0.05% Alcalase 2.4L FG was added to the fermentation broth.

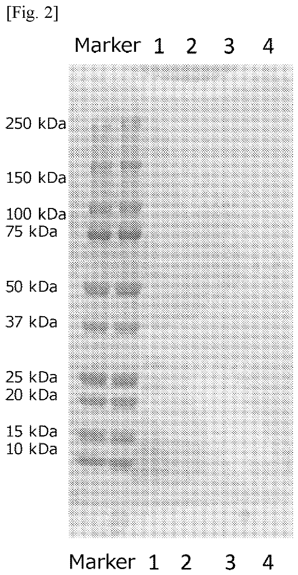

The results of SDS-PAGE are shown in Figure 2. In Fig. 2, lane 1 corresponds to Alcalase 2.4L FG (whose protein concentration was adjusted to about 0.8 mg-as BSA/mL), lane 2 corresponds to the sample without Alcalase 2.4L FG treatment, lane 3 corresponds to the sample treated with 0.05% Alcalase 2.4L FG, and lane 4 corresponds to the sample treated with 0.5% Alcalase 2.4L FG. Degradation of proteins (especially those having a molecular weight of 10 to 15 kDa) in the fermentation broth was observed in the samples treated with Alcalase 2.4L FG (lanes 3 to 4).

From the above, it was revealed that by treating a fermentation broth containing vanillin with a protease and then subjecting it to solvent extraction using an organic solvent, emulsification during the solvent extraction can be prevented. Specifically, degradation of proteins (especially those having a molecular weight of 10 to 15 kDa) in the fermentation broth may prevent emulsification during the solvent extraction.

<2> Evaluation of emulsification-preventing effect of protease (2)

In this example, a fermentation broth containing vanillin was treated with a protease and then subjected to solvent extraction using an organic solvent, to thereby evaluate the emulsification-preventing effect of the protease treatment. As the protease, Alcalase 2.4L FG (derived from *Bacillus licheniformis,* Novozymes Japan) was used. As the organic solvent, ethyl acetate and butyl acetate each were used.

The 1612BP heat-aggregated broth was filtered under reduced pressure, to thereby obtain a cell filtrate. The obtained cell filtrate was concentrated, to thereby obtain a concentrated filtrate, wherein the concentration rate was 1.70 wt/wt-fold in terms of the concentration rate to the culture broth. Protease treatment was carried out with addition of 0.1% Alcalase 2.4L FG using 5 mL of the concentrated filtrate in the same manner as Example <1>, then solvent extraction was carried out with addition of 5 mL ethyl acetate or 5 mL butyl acetate, and then the degree of emulsification was observed.

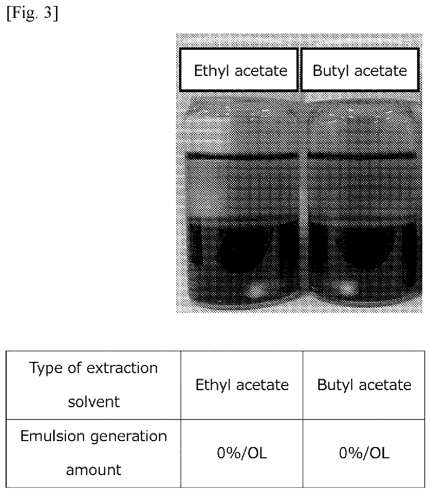

The results are shown in Figure 3. No emulsification was observed when the solvent extraction was carried out with ethyl acetate or butyl acetate. It was suggested that by treating a fermentation broth containing vanillin with a protease and then subjecting it to solvent extraction using an organic solvent, emulsification during the solvent extraction can be prevented regardless of the type of the organic solvent.

<3> Evaluation of emulsification-preventing effect of protease (3)

In this example, a fermentation broth containing vanillin was treated with a protease and then subjected to solvent extraction using an organic solvent, to thereby evaluate the emulsification-preventing effect of the protease treatment. As the protease, various proteases shown in Table 1 each were used. A summary of the catalog specifications of those proteases is shown in Table 2. As the organic solvent, butyl acetate was used.

*Corynebacterium glutamicum* introduced with the aromatic carboxylic acid reductase (ACAR) derived from *Gordonia effusa* (WO2018/079705) was cultured in a culture medium containing vanillic acid, to thereby obtain a culture broth containing 9.4 g/L vanillin. The culture broth was adjusted to pH3 by addition of 98% sulfuric acid and kept at 50°C for 30 minutes, to thereby obtain a sterilized broth. The sterilized broth was kept at 120°C for 3 minutes, to thereby obtain a heat-aggregated broth. The heat-aggregated broth was filtered using a filter press, and thereby separated into solids such as bacterial cells and a filtrate. The obtained solids were washed with water in a volume equal to the heat-aggregated broth, and then filtered again. The washing process was carried out again. The filtrate from the three times was mixed, to thereby obtain a cell-removed broth. The obtained cell-removed broth was designated as "1706BP cell-removed broth".

The 1706BP cell-removed broth was concentrated to 1.76 wt/wt-fold in terms of the concentration rate to the culture broth, to thereby obtain a concentrated cell-removed broth. The obtained concentrated cell-removed broth (5 mL) was adjusted to pH 7.0, then added with 0.1 wt% of each protease, and stirred at 40°C for 1 hour. Next, the reaction mixture was adjusted to pH 6.0, then added with an equal volume of butyl acetate, and stirred vigorously using a stirrer. One hour later, stirring was stopped, the mixture was allowed to stand for about 5 minutes, and then the degree of emulsification was observed.

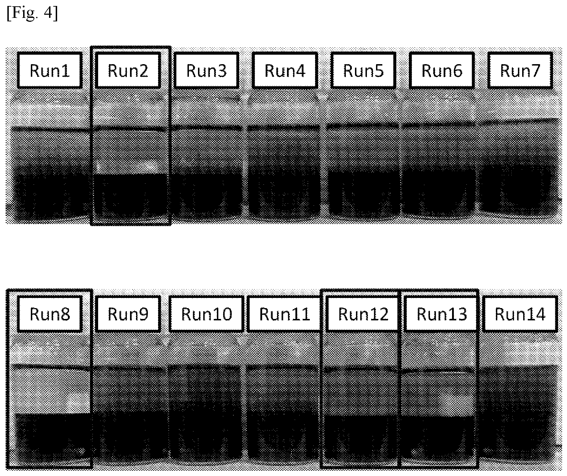

The results are shown in Tables 1 to 2 and Figure 4. Some proteases showed the emulsification-preventing effect but some proteases did not show the emulsification-preventing effect. A relationship between the origin of proteases and the presence or absence of the emulsification-preventing effect was observed, and all the proteases derived from the genus Bacillus showed the emulsification-preventing effect. On the other hand, no clear relationship between the properties of proteases other than their origin and the presence or absence of the emulsification-preventing effect was observed.

Table 1

|  | Enzyme name | Emulsion generation amount |
|---|---|---|
| Run 1 | No addition | 100%/OL |
| Run 2 | Alcalase 2.4L FG | 0%/OL |
| Run 3 | Protease A "Amano" SD | 60%/OL |
| Run 4 | Protease M "Amano" SD | 100%/OL |
| Run 5 | Protease P "Amano" 3SD | 80%/OL |
| Run 6 | Papain W-40 | 85%/OL |
| Run 7 | Peptidase R | 100%/OL |
| Run 8 | ProteAX | 0%/OL |
| Run 9 | Thermoase PC10F | 100%/OL |
| Run 10 | Bromelain F | 100%/OL |
| Run 11 | Pancreatin F | 100%/OL |
| Run 12 | Protin SD-AY10 | 0%/OL |
| Run 13 | Protin SD-NY10 | 0%/OL |
| Run 14 | Neurase F3G | 100%/OL |

Table 2

| | Enzyme name | Type | Digestion type | Origin |
|---|---|---|---|---|
| 1 | No addition | − | − | − |
| 2 | Alcalase 2.4L FG | Alkaline protease | Endo | Bacillus licheniformis |
| 3 | Protease A "Amano" SD | Neutral protease | Endo + Exo | Aspergillus sp. |
| 4 | Protease M "Amano" SD | Acidic protease | Endo + Exo | Aspergillus sp. |
| 5 | Protease P "Amano" 3SD | Neutral protease | Endo + Exo | Aspergillus sp. |
| 6 | Papain W-40 | Neutral protease | Endo | Papaya |
| 7 | Peptidase R | Neutral peptidase | Endo + Exo | Rhizopus sp. |
| 8 | ProteAX | Neutral peptidase | Endo + Exo | Aspergillus oryzae |

| 9 | Thermoase PC10F | Thermostable metalloprotease | Endo | Geobacillus sp. |
|---|---|---|---|---|
| 10 | Bromelain F | Alkaline protease | Endo | Pineapple |
| 11 | Pancreatin F | protease + amylase | Endo + Exo | Pig pancreas |
| 12 | Protin SD-AY10 | Alkaline serine protease | Endo | Bacillus sp. |
| 13 | Protin SD-NY10 | Neutral metalloprotease | Endo | Bacillus sp. |
| 14 | Neurase F3G | Acidic protease + lipase | Endo | Rhizopus sp. |

Table 2 (continued)

| | Titer | Target site | Optimal pH | Optimal temperature | Initial velocity | Size of degradation product |
|---|---|---|---|---|---|---|
| 1 | – | – | – | – | – | – |
| 2 | 2.4 AU-A/g | Ser | 7-9 | 30-65°C | ? | ? |
| 3 | 50,000 U/g (Folin method) | Gln, Met, Cys | 7.0 | 50°C | Slow | Small |
| 4 | 40,000 U/g (Folin method) | Gln, Ser, Met, Cys | 6.0 | 50°C | Slow | Extremely Small |
| 5 | 300,000 U/g (Folin method) | Alg, Gln, Thr | 8.0 | 45°C | Slow | Small |
| 6 | 400,000 U/g (Japan's specifications and standards) | Alg, Gln, Thr | 8.0-9.0 | 80°C | Slow | Large |
| 7 | 420 U/g (LGG method) | Arg | 8.0 | 45°C | Slow | Extremely small |
| 8 | 1,400 U/g (LNA method) | Gln, Ser, Thr, Met | 7.0 | 50°C | Slow | Extremely small |
| 9 | 90,000 U/g (Folin | Arg | 8.0 | 70°C | Fast | Large |

| | | | | | | |
|---|---|---|---|---|---|---|
| | | method) | | | | | | |
| 10 | 800,000 U/g (Japan's specifications and standards) | ? | 9.0 | 70°C | Fast | Large |
| 11 | 28,000 U/g (Japanese Pharmacopoeia) | ? | 9.0 | 45°C | ? | ? |
| 12 | 80,000 U/g (Folin method) | Lys, Leu | 10.0 | 60°C | Fast | Large |
| 13 | 70,000 U/g (Folin method) | Thr, Cys | 7.0 | 55°C | Fast | Large |
| 14 | 40,000 U/g (Folin method) | ? | 3.0 | 45°C | Slow | Large |

<4> Evaluation of recovery rate of vanillin and organic solvent

In this example, the emulsification-preventing effect of the protease treatment and the recovery rate of vanillin and an organic solvent were evaluated in a scaled-up system. As the protease, Alcalase 2.4L FG (derived from *Bacillus licheniformis,* Novozymes Japan) was used. As the organic solvent, ethyl acetate and butyl acetate each were used.

The 1706BP cell-removed broth was concentrated to 1.76 wt/wt-fold in terms of the concentration rate to the culture broth, to thereby obtain a concentrated cell-removed broth. The concentrated cell-removed broth (80 mL) was adjusted to pH 7.5, then added with 0.1 wt% of Alcalase 2.4L FG, and stirred at 40°C for 1 hour. Next, the reaction mixture was added with an equal volume of ethyl acetate or butyl acetate, and stirred vigorously using a stirrer. One hour later, stirring was stopped, and then the degree of emulsification was observed. The aqueous layer and the organic layer were separated using a separating funnel, and the recovery rate of vanillin and the organic solvents was evaluated.

The results are shown in Table 3. As with the case of 5-mL scale (Example <2>), the emulsion generation amount was 0%/OL, and that is, the emulsification-preventing effect was observed also in this scale. The recovery rate of vanillin was more than 95% in one extraction in both solvents. Due to the difference in solubility in water, the recovery rate of ethyl acetate in the organic layer was about 95%, whereas the recovery rate of butyl acetate in the organic layer was more than 99%.

Table 3

| | Ethyl acetate | Butyl acetate |
|---|---|---|
| Vanillin recovery rate | 95.7% | 96.6% |
| Organic solvent recovery rate | 94.7% | 99.7% |

<5> Evaluation of emulsification-preventing effect of enzyme other than protease

In this example, a fermentation broth containing vanillin was treated with an enzyme other than protease and then subjected to solvent extraction using an organic solvent, to thereby evaluate the emulsification-preventing effect of the treatment with the enzyme other than protease. As the enzyme, various enzymes shown in Table 4 each were used. As the organic solvent, butyl acetate was used.

The 1706BP cell-removed broth was concentrated to 1.76 wt/wt-fold in terms of the concentration rate to the culture broth, to thereby obtain a concentrated cell-removed broth. The obtained concentrated cell-removed broth (5 mL) was adjusted to pH 7.0, then added with 0.1 wt% of each enzyme, and stirred at 40°C for 1 hour. Next, the reaction mixture was adjusted to pH 6.0, then added with an equal volume of butyl acetate, and stirred vigorously using a stirrer. One hour later, stirring was stopped, the mixture was allowed to stand for about 5 minutes, and then the degree of emulsification was observed.

The results are shown in Table 4. The emulsion generation amount was 100%/OL in the case of using any of the enzymes shown in Table 4, and that is, no emulsification-preventing effect was observed. From the above, there was suggested a possibility that contaminated proteins in the fermentation broth may specifically contribute to emulsification during the solvent extraction.

Table 4

| | | Enzyme name |
|---|---|---|
| | Run 1 | Pectinase G "Amano" |
| | Run 2 | Pectinase PL "Amano" |
| | Run 3 | Lipase A "Amano" 6 |
| | Run 4 | Lipase GS "Amano" 250G |
| | Run 5 | Lipase AY "Amano" 30SD |
| | Run 6 | Pullulanase "Amano" 3 |
| | Run 7 | Hemicellulase "Amano" 90 |
| | Run 8 | Cellulase T "Amano" 4 |
| | Run 9 | Cellulase A "Amano" 3 |
| | Run 10 | Dizyme GPK (Pullulanase and Glucoamylase) |
| | Run 11 | Kleistase PL45 (Pullulanase) |
| | Run 12 | Alpha-glucosidase "Amano" |

<6> Evaluation of effect of agitation power during solvent extraction (1)

In this example, an effect of agitation power upon collecting vanillin from a fermentation broth containing vanillin by solvent extraction using an organic solvent was evaluated. As the organic solvent, toluene was used.

*Corynebacterium glutamicum* introduced with the aromatic carboxylic acid reductase (ACAR) derived from *Gordonia effusa* (WO2018/079705) was cultured in a culture medium containing vanillic acid, to thereby obtain a culture broth containing 5.2 g/L, 9.4 g/L, or 11 g/L vanillin. The culture broth was adjusted to pH3 by addition of 98% sulfuric acid and kept at 50°C for 30 minutes, to thereby obtain a sterilized broth. The sterilized broth was kept at 120°C for 3 minutes, to thereby obtain a heat-aggregated broth.

The heat-aggregated broth (500 mL) was added to a separating funnel having a jacket (inner diameter 110 mm) and adjusted to pH 6.0. Toluene (500 mL) was gently added thereto, and then warm water was passed through the jacket to heat the internal temperature to 70°C. Stirring was carried out using retreating blades (four blades, blade diameter 90 mm, blade width 10 mm), anchor blades (four blades, blade diameter 90 mm, blade width 45 mm), or half-moon paddle blades (two blades, blade diameter 90 mm, blade width 20 mm) at a stirring speed at which the liquid-liquid interface (i.e. the interface between the aqueous layer and the organic layer) was maintained, and vanillin concentrations in the aqueous and organic layers were analyzed over time. In the case of the retreating blades, the turbulence of the liquid-liquid interface became more pronounced when the stirring speed was higher than 120 rpm (equivalent to 27.4 W/kL). 24 hours later, the aqueous and organic layers were separated and the yield was calculated from the weight and the vanillin concentration of each layer. The time when the vanillin concentration in the organic layer reached equilibrium was considered as the extraction time.

The agitation power was calculated using Nagata's formula.

The mass transfer coefficient was calculated according to the following procedure.

Assuming that the rate-limiting step of extraction is the mass transfer at the liquid-liquid interface, the mass transfer coefficient k was calculated using the following formula.

$$J = k \cdot \Delta C \;\ldots\ldots\; (1)$$

J: Mass transfer flux [$g/m^2/s$]
k: Mass transfer coefficient [m/s]

ΔC: Difference in concentration [g/m$^3$]

$$\Delta C = C_{water} \cdot P - C_{tol} \quad \ldots\ldots (2)$$

$C_{water}$: Vanillin concentration in aqueous layer [g/m$^3$]
$C_{tol}$: Vanillin concentration in toluene layer [g/m$^3$]
P: Distribution coefficient

$$k = D / \delta \quad \ldots\ldots (3)$$

D: Diffusion coefficient [m$^2$/s]
δ: Thickness of boundary film [m]

From Formula (1), the mass transfer at every 1 min was calculated from the interface area, the liquid volume, and the initial concentration on Excel, and the concentration was calculated. The mass transfer coefficient k, which minimizes the sum of the squares of the differences between the calculated and measured concentrations at each time point, was calculated using a solver.

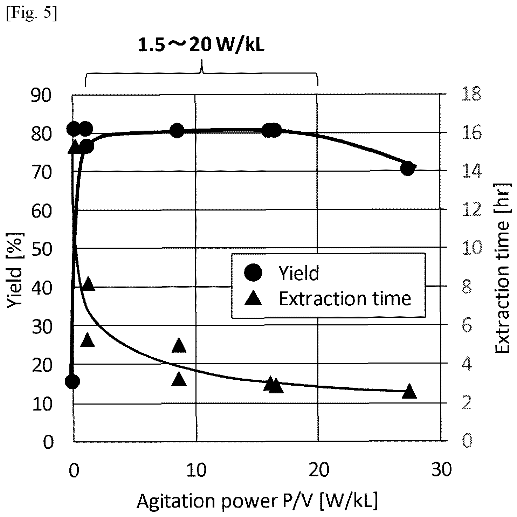

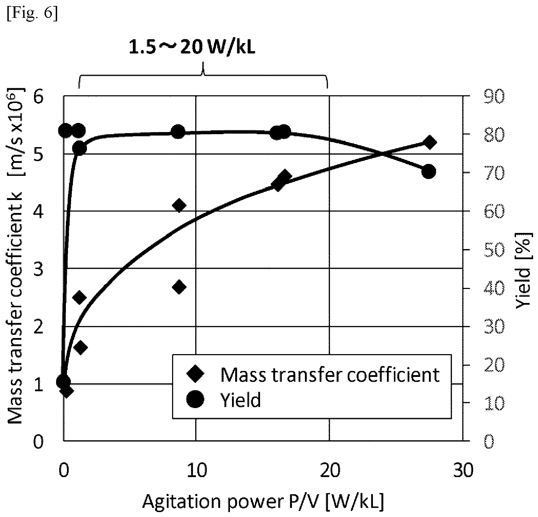

The results are shown in Table 5 and Figures 5 to 6. By adjusting the agitation power during the solvent extraction so that the liquid-liquid interface (i.e. the interface between the aqueous layer and the organic layer) was maintained, emulsification was able to be prevented, and vanillin was able to be extracted in high yield. Particularly, favorable results were obtained when the agitation power was 1.1 to 16.6 W/kL. In addition, from Figs. 5 to 6, a preferred range of agitation power can be assumed to be, for example, 1.5 to 20 W/kL.

Table 5 Results of vanillin extraction from fermentation broth using toluene

| Agitation power [W/kL] | Mass transfer coefficient [m/s $\times 10^6$] | Yield [%] | Extraction time [hr] | Blade tip speed [m/s] | Type of stirring blade |
|---|---|---|---|---|---|
| 0.0 | - | 15.4 | - | 0 | - |
| 0.2 | 0.9 | 80.7 | 15.3 | 0.09 | Anchor blade |
| 1.1 | 2.5 | 80.7 | 5.3 | 0.18 | Half-moon paddle |
| 1.3 | 1.6 | 76.3 | 8.2 | 0.18 | Anchor blade |
| 8.6 | 2.7 | 80.3 | 5.0 | 0.37 | Retreating blade |
| 8.6 | 4.1 | - | 3.3 | 0.37 | Half-moon paddle |
| 16.1 | 4.5 | 80.2 | 3.0 | 0.47 | Retreating blade |
| 16.6 | 4.6 | 80.4 | 2.9 | 0.47 | Retreating blade |
| 27.4 | 5.2 | 70.2 | 2.6 | 0.57 | Retreating blade |

<7> Evaluation of effect of agitation power during solvent extraction (2)

In this example, an effect of agitation power upon collecting vanillin from a fermentation broth containing vanillin by solvent extraction using an organic solvent was evaluated. As the organic solvent, ethyl acetate was used.

*Corynebacterium glutamicum* introduced with the aromatic carboxylic acid reductase (ACAR) derived from *Gordonia effusa* (WO2018/079705) was cultured in a culture medium containing vanillic acid, to thereby obtain a culture broth containing 11 g/L vanillin. The culture broth was adjusted to pH3 by addition of 98% sulfuric acid and kept at 50°C for 30 minutes, to thereby obtain a sterilized broth.

The sterilized broth (500 mL) was added to a separating funnel having a jacket (inner diameter 110 mm) and adjusted to pH 6.0. Ethyl acetate (500 mL) was gently added thereto, and then warm water was passed through the jacket to heat the internal temperature to 60°C. Stirring was carried out using retreating blades (four blades, blade diameter 90 mm, blade width 10 mm) at a stirring speed shown in Table 6, and vanillin concentrations in the aqueous and organic layers were analyzed over time. 24 hours later, the aqueous and organic layers were separated and the yield was calculated from the weight and the vanillin concentration of each layer.

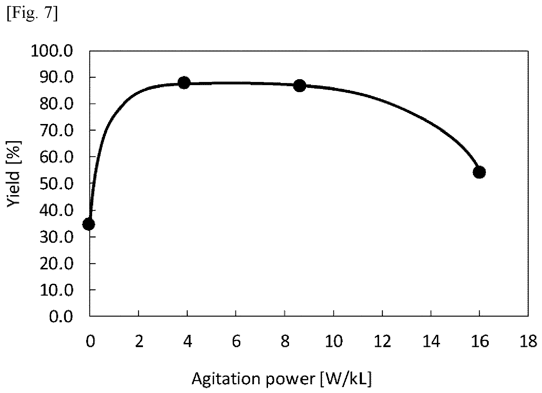

The results are shown in Table 6 and Figure 7. Also in the case of using ethyl acetate as the organic solvent, by adjusting the agitation power during the solvent extraction so that the liquid-liquid interface (i.e. the interface between the aqueous layer and the organic layer) was maintained, emulsification was able to be prevented, and vanillin was able to be extracted in high yield (3.89 W/kL or 8.64 W/kL). In addition, from Fig. 7, a preferred range of agitation power can be assumed to be, for example, 1.5 to 10 W/kL.

Table 6 Results of vanillin extraction from fermentation broth using ethyl acetate

| Agitation power [W/KL] | Agitation power [%] | Agitation power [m/s] | Type of stirring blade |
|---|---|---|---|
| 0 | 34.4 | 0 | Retreating blade |
| 3.89 | 87.5 | 0.28 | Retreating blade |
| 8.64 | 86.3 | 0.38 | Retreating blade |
| 16.02 | 53.8 | 0.47 | Retreating blade |

Industrial Applicability

According to the present invention, emulsification upon extracting an objective substance such as vanillin with an organic solvent from a fermentation broth can be prevented.

**Claims**

1. A method for producing an objective substance, the method comprising the following steps (1A) and (1B):

   (1A) a step of treating a fermentation broth containing the objective substance with a protease; and
   (1B) a step of extracting the objective substance with an organic solvent from the fermentation broth after the treatment,
   wherein the protease is one or more kinds of proteases selected from proteases derived from *Bacillus* bacteria and proteases derived from *Aspergillus* fungi.

2. The method according to claim 1, wherein the objective substance is an aromatic aldehyde.

3. The method according to claim 1 or 2, wherein the objective substance is vanillin.

4. The method according to any one of claims 1 to 3, provided that cases where the protease is Protease M "Amano" SD, Protease A "Amano" SD, or Protease P "Amano" 3SD are excluded.

5. The method according to any one of claims 1 to 4, wherein the protease is one or more kinds of proteases selected from serine proteases and metalloproteases.

6. The method according to any one of claims 1 to 5, wherein the protease is one or more kinds of proteases selected from subtilisin and bacillolysin.

7. The method according to any one of claims 1 to 6, wherein the protease is one or more kinds of proteases selected from proteases derived from *Bacillus licheniformis,* proteases derived from *Bacillus amyloliquefaciens,* and proteases derived from *Aspergillus oryzae.*

8. The method according to any one of claims 1 to 7, wherein the protease is one or more kinds of proteases selected from Alcalase (registered trademark) 2.4L FG, Protin SD-NY10, Protin SD-AY10, and ProteAX (registered trademark).

9. The method according to any one of claims 1 to 8, wherein the use amount of the protease is 10 to 500,000 U in terms of the protease activity measured by the Folin method, 0.2 to 10,000 U in terms of the protease activity measured by the LNA method, or 0.5 to 20,000 mAU in terms of the protease activity measured by the Anson method, to 100 mL of the fermentation broth.

10. The method according to any one of claims 1 to 9, wherein the organic solvent is one or more kinds of components selected from toluene, ethyl acetate, and butyl acetate.

11. The method according to any one of claims 1 to 10, wherein the fermentation broth in the step (1A) is a fermentation broth after cell removal.

12. The method according to any one of claims 1 to 11, wherein the step (1B) is carried out under stirring conditions.

13. The method according to any one of claims 1 to 12, wherein the method further comprises, prior to the step (1A), a step of generating the objective substance using a microorganism having an objective substance-producing ability, to thereby obtain the fermentation broth.

14. The method according to any one of claims 1 to 13, wherein the method further comprises, after the step (1B), collecting an organic layer containing the objective substance.

15. The method according to claim 14, wherein the method further comprises purifying the objective substance from the organic layer.

16. A method for producing an objective substance, the method comprising the following step (2A):
(2A) a step of extracting the objective substance with an organic solvent from a fermentation broth containing the objective substance,
wherein the step (2A) is carried out by stirring the fermentation broth and the organic solvent with an adjusted agitation power.

17. The method according to claim 16, wherein the objective substance is an aromatic aldehyde.

18. The method according to claim 16 or 17, wherein the objective substance is vanillin.

19. The method according to any one of claims 16 to 18, wherein the adjusted agitation power is an agitation power with which a liquid-liquid interface is maintained.

20. The method according to any one of claims 16 to 19, wherein the adjusted agitation power is more than 0 and 20 W/kL or less.

21. The method according to any one of claims 16 to 20, wherein the adjusted agitation power is more than 0 and 15 W/kL or less.

22. The method according to any one of claims 16 to 21, wherein the adjusted agitation power is more than 0 and 10 W/kL or less.

23. The method according to any one of claims 16 to 22, wherein the adjusted agitation power is 1.5 W/kL or more.

24. The method according to any one of claims 16 to 23, wherein the organic solvent is one or more kinds of components selected from toluene, ethyl acetate, and butyl acetate.

25. The method according to any one of claims 16 to 24, wherein the organic solvent is toluene, and the adjusted agitation power is more than 0 and 20 W/kL or less.

26. The method according to any one of claims 16 to 24, wherein the organic solvent is ethyl acetate, and the adjusted agitation power is more than 0 and 10 W/kL or less.

27. The method according to any one of claims 16 to 26, wherein the fermentation broth is a fermentation broth containing cells.

28. The method according to any one of claims 16 to 27, wherein the method further comprises, prior to the step (2A), a step of generating the objective substance using a microorganism having an objective substance-producing ability, to thereby obtain the fermentation broth.

29. The method according to any one of claims 16 to 28, wherein the method further comprises, after the step (2A), collecting an organic layer containing the objective substance.

30. The method according to claim 29, wherein the method further comprises purifying the objective substance from the organic layer.

[Fig. 1]

| Addition amount of Alcalase 2.4L FG | 0% | 0.05% | 0.1% | 0.5% | 1% |
|---|---|---|---|---|---|
| Emulsion generation amount | 100%/OL | 0%/OL | 0%/OL | 0%/OL | 0%/OL |

[Fig. 2]

[Fig. 3]

| Type of extraction solvent | Ethyl acetate | Butyl acetate |
|---|---|---|
| Emulsion generation amount | 0%/OL | 0%/OL |

[Fig. 4]

[Fig. 5]

[Fig. 6]

[Fig. 7]

**INTERNATIONAL SEARCH REPORT**

| International application No. |
|---|
| PCT/JP2020/017952 |

**A. CLASSIFICATION OF SUBJECT MATTER**
C07C 47/058(2006.01)i; C07C 47/58(2006.01)i; C12P 7/22(2006.01)i; C12P 7/24(2006.01)i; C12N 1/00(2006.01)i
FI: C12P7/24; C07C47/58; C07C47/058 A; C12P7/22; C12N1/00 K
According to International Patent Classification (IPC) or to both national classification and IPC

**B. FIELDS SEARCHED**

Minimum documentation searched (classification system followed by classification symbols)
C07C47/058; C07C47/58; C12P7/22; C12P7/24; C12N1/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
|---|---|---|
| X | CN 103709021 A (ZHEJIANG APELOA BIO PHARMACEUTICAL CO., LTD.) 09.04.2014 (2014-04-09) claims, paragraph [0003], example 1 | 16-24, 26-30 |
| Y | claims, paragraph [0003], example 1 | 1-15, 25, 27-30 |
| Y | JP 2017-521084 A (ROQUETTE FRERES) 03.08.2017 (2017-08-03) paragraphs [0054], [0055], example 2 | 1-15 |
| Y | JP 55-079337 A (YHTYNEET PAPERITEHTAAT OY) 14.06.1980 (1980-06-14) claims | 25, 27-30 |

☐ Further documents are listed in the continuation of Box C.  ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 08 July 2020 (08.07.2020) | 28 July 2020 (28.07.2020) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

INTERNATIONAL SEARCH REPORT

Information on patent family members

| International application No. |
| --- |
| PCT/JP2020/017952 |

| Patent Documents referred in the Report | Publication Date | Patent Family | Publication Date |
| --- | --- | --- | --- |
| CN 103709021 A | 09 Apr. 2014 | (Family: none) | |
| JP 2017-521084 A | 03 Aug. 2017 | US 2017/0137477 A1 paragraphs [0075]-[0078], example 2 WO 2016/009146 A1 entire text EP 3169696 A1 entire text KR 10-2017-0023065 A entire text CN 106661082 A entire text | |
| JP 55-079337 A | 14 Jun. 1980 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2011177159 A
- WO 2010114552 A
- EP 0952221 A
- WO 2018079687 A
- WO 2018079686 A
- WO 2018079685 A
- WO 2018079684 A
- WO 2018079683 A
- WO 2017073701 A
- WO 2018079705 A
- WO 2017122747 A
- WO 2013022881 A1
- WO 2013022881 A
- WO 2018079705 A1
- JP 2109985 A, Kokai
- US 5882888 A
- EP 805867 B1
- JP 3210184 A, Kokai
- JP 2072876 A, Kokai
- US 5185262 A
- JP 1191686 A, Kokai
- JP 58192900 A, Kokai
- JP 57134500 A, Kokai
- JP 58035197 A, Kokai
- JP 57183799 A, Kokai
- US 6090597 A
- JP 9322774 A, Kokai
- JP 10215883 A, Kokai
- WO 2007046389 A
- WO 2013069634 A
- JP 9070291 A, Kokai
- WO 2005010175 A
- WO 0018935 A
- RU 2006134574, Katashkina JI
- JP 2207791 A, Kokai
- US 6303383 B
- JP 5007491 A, Kokai
- JP 7075589 A, Kokai
- JP 5244941 A, Kokai
- JP 2007104942 A, Kokai
- JP 2010207094 A, Kokai
- JP 50089592 A, Kokai
- US 5272073 A

### Non-patent literature cited in the description

- Derivations and Genotypes of some mutant derivatives of Escherichia coli K-12. **NEIDHARDT ; BACKMANN B.J. et al.** Escherichia coli and Salmonella Cellular and Molecular Biology. American Society for Microbiology Press, 1996, 2460-2488
- *Biotechnol. Bioeng.,* 27 March 2007, vol. 98 (2), 340-348
- *Int. J. Syst. Bacteriol.,* 1989, vol. 39, 337-345
- *Int. J. Syst. Bacteriol.,* 1993, vol. 43, 162-173
- *Int. J. Syst. Bacteriol.,* 1991, vol. 41, 255
- *Int. J. Syst. Evol. Microbiol.,* 2010, vol. 60, 874-879
- *International Journal of Systematic and Evolutionary Microbiology,* 2007, vol. 57, 1828-1833
- *J. Biol. Chem.,* 2007, vol. 282 (1), 478-485
- *App. Env. Microbiol.,* 2009, vol. 75 (9), 2765-2774
- **M.T. CHAUDHRY et al.** *Microbiology,* 2007, vol. 153, 857-865
- **M. T. CHAUDHRY et al.** *Microbiology,* 2007, vol. 153, 857-865
- *Current Microbiology,* 2005, vol. 51, 59-65
- *Appl. Microbiol. Biotechnol.,* 2012, vol. 95, 77-89
- **KUNJAPUR AM et al.** *J. Am. Chem. Soc.,* 2014, vol. 1 (136), 11644-11654
- **HANSEN EH et al.** *App. Env. Microbiol.,* 2009, vol. 75, 2765-2774
- *J Bacteriol,* 2001, vol. 183, 3276-3281
- *Meth. Enz.,* 1970, vol. 17A, 526-529
- *J Mol Microbiol Biotechnol,* 2008, vol. 15, 277-286
- **MILLER, J.H.** Experiments in Molecular Genetics. Cold Spring Harbor Laboratory, 1972
- **DATSENKO, K.A. ; WANNER, B.L.** *Proc. Natl. Acad. Sci. USA,* 2000, vol. 97, 6640-6645
- *Agric. Biol. Chem.,* 1984, vol. 48, 2901-2903
- Fundamental Microbiology. Genetic Engineering. KYORITSU SHUPPAN CO., LTD, 1987, vol. 8

- *Gene,* 1987, vol. 60 (1), 115-127
- **HIGUCHI, R.** PCR Technology. Stockton Press, 1989, 61
- **CARTER, P.** *Meth. in Enzymol.,* 1987, vol. 154, 382
- **KRAMER, W. ; FRITS, H.J.** *Meth. in Enzymol.,* 1987, vol. 154, 350
- **KUNKEL, T.A. et al.** *Meth. in Enzymol.,* 1987, vol. 154, 367
- *Appl. Microbiol. Biotechnol.,* 2000, vol. 53, 674-679
- *Journal of Biotechnology,* 2003, vol. 104, 311-323
- *Appl. Environ. Microbiol.,* December 2005, vol. 71 (12), 8587-96
- **GOLDSTEIN et al.** Prokaryotic Promoters in Biotechnology. *Biotechnol. Annu. Rev.,* 1995, vol. 1, 105-128
- **OLINS P.O. et al.** *Gene,* 1988, vol. 73, 227-235
- **NAKAMURA, Y et al.** Codon Usage Database. *Nucl. Acids Res.,* 2000, vol. 28, 292, www.kazusa.or.jp/codon
- **MANDEL, M. ; HIGA, A.** *J. Mol. Biol.,* 1970, vol. 53, 159-162
- **DUNCAN, C.H. ; WILSON, G.A. ; YOUNG, F.E.** *Gene,* 1997, vol. 1, 153-167
- **CHANG, S. ; CHOEN, S.N.** *Mol. Gen. Genet.,* 1979, vol. 168, 111-115
- **BIBB, M.J. ; WARD, J.M. ; HOPWOOD, O.A.** *Nature,* 1978, vol. 274, 398-400
- **HINNEN, A. ; HICKS, J.B. ; FINK, G.R.** *Proc. Natl. Acad. Sci. USA,* 1978, vol. 75, 1929-1933
- **SAMBROOK, J. et al.** Molecular Cloning A Laboratory Manual. Cold Spring Harbor Laboratory Press, 2001
- Molecular Cloning. Cold Spring Harbor Laboratory Press, 2001
- *Journal of Biological Chemistry,* 1997, vol. 272, 8611-8617
- *Proceedings of the National Academy of Sciences, USA,* 1998, vol. 95, 5511-5515
- *Journal of Biological Chemistry,* 1991, vol. 26 (116), 20833-20839
- **DATSENKO, K.A ; WANNER, B.L.** *Proc. Natl. Acad. Sci. USA,* 2000, vol. 97, 6640-6645
- **CHO, E.H. ; GUMPORT, R.I. ; GARDNER, J.F.** *J. Bacteriol.,* 2002, vol. 184, 5200-5203
- *J. Am. CHm. Soc.,* 1998, vol. 120
- *J. App. Microbiol.,* 2013, vol. 116, 903-910
- **HORI, H. et al.** *Appl. Microbiol. Biotechnol.,* 1997, vol. 48 (6), 693-698
- **YAMAMOTO, S et al.** *Biosci. Biotechnol. Biochem.,* 2005, vol. 69 (4), 784-789
- **C. AUG'E ; CH. GAUTHERON.** *Tetrahedron Lett.,* 1988, vol. 29, 789-790
- **MURATA, K. et al.** *Agric. Biol. Chem.,* 1988, vol. 52 (6), 1471-1477